# EUROPÄISCHE PATENTSCHRIFT

(11) **EP 1 488 768 B1**
(45) Veröffentlichungstag und Bekanntmachung des Hinweises auf die Patenterteilung: **06.12.2006**
(21) Anmeldenummer: 04013965.1
(22) Anmeldetag: 15.06.2004
(51) Int. Cl.: A61F 9/02, G02C 7/10, G02C 7/16, G02B 27/01, A61N 5/06

(54) **Augenschutz gegen Strahlung**
Eye protecting device against radiation
Dispositif de protection de radiations pour les yeux

(30) Priorität: 16.06.2003 DE 10327017
(43) Veröffentlichungstag der Anmeldung: 22.12.2004
(73) Patentinhaber: JK-Holding GmbH, 53578 Windhagen (DE)
(72) Erfinder: Kratz, Walter, 53783 Eitorf (DE)
(74) Vertreter: Koepe, Gerd L.

(56) Entgegenhaltungen:
- EP-A- 0 612 539
- EP-A- 1 219 321
- US-A- 4 154 513
- US-A- 4 933 755
- US-A1- 2003 011 673

## Beschreibung

Die vorliegende Erfindung betrifft einen Augenschutz gegen Strahlung. Insbesondere betrifft die Erfindung eine Schutzvorrichtung gegen UV-Strahlung und/oder sichtbare Strahlung, wie sie von natürlichen oder künstlichen UV-Strahlungsquellen wie beispielsweise von der Sonne oder von Röhren von UV-Bräunungsgeräten abgestrahlt wird, für die Augen von Menschen, die sich der natürlichen UV-Strahlung (z. B. der Sonne) aussetzen, oder für die Augen der Benutzer solcher UV-Bräunungsgeräte.

Bräunungsgeräte auf der Basis von UV-Strahlern sind in unterschiedlichen Ausbildungen und Größen bekannt. Bräunungsgeräte zur Ganzkörper-Bräunung weisen als Unterteil eine gegebenenfalls mit einem Profil versehene Liege ("Bank") auf, die unterhalb einer für die bräunende Strahlung transparenten Oberfläche mit die bräunende Strahlung abstrahlenden UV-Strahlungsquellen wie z. B. UV-Strahler-Röhren bestückt ist. Dem Unterteil der Bräunungsgeräte entspricht ein bewegliches Oberteil, das entweder um eine parallel zu einer Kante, üblicherweise parallel zu einer der beiden längeren Kanten, der Liege verlaufende Achse schwenkbar oder an einem Stativ oder an einer Aufhängung an einer Raumdecke in vertikaler Richtung bewegbar ist. Das Oberteil ist - oberhalb einer für die bräunende Strahlung transparenten unteren Fläche - ebenfalls mit die bräunende Strahlung abstrahlenden UV-Strahlungsquellen wie beispielsweise UV-Strahler-Röhren bestückt. Benutzer eines Bräunungsgerätes heben oder schwenken das Oberteil in die offene Position, die ein bequemes "Einsteigen" ermöglicht, legen sich auf die Liege, schließen das Oberteil und schalten dann die UV-Strahler-Röhren für eine vorgewählte Bräunungszeit ein, die im Bereich weniger Minuten, jedoch durchaus auch bei 15 Minuten oder länger liegen kann.

Zwar ist die von den UV-Stahlungsquellen wie den UV-Strahler-Röhren abgestrahlte Strahlung für die Haut vergleichsweise ungefährlich, insbesondere für die UV-Strahlung gewohnte oder durch geeignete Schutzfilme (Sonnencreme, Sonnenöl) geschützte Haut; die Augen des Benutzers können jedoch bei direktem Kontakt mit intensiver UV-Strahlung bereits nach kurzer Exposition beeinträchtigt werden. Deswegen ist es bei Benutzung von UV-Bräunungsgeräten vorgeschrieben, einen geeigneten, für die UV-Strahlung (wie auch für den überwiegenden Anteil der sichtbaren Strahlung) undurchlässigen, im wesentlichen die durch oberes und unteres Lid gebildeten Flächen der Augenhöhle abdeckenden Augenschutz zu tragen.

Viele Benutzer verstoßen jedoch gegen die zu ihrem Schutz erlassene Vorschrift und - statt einen Augenschutz zu tragen - schließen beim Bräunen einfach die Augen, zum einen aufgrund der Tatsache, daß sie das Tragen des Augenschutzes als unbequem empfinden, zum anderen deswegen, weil beim Tragen eines Augenschutzes beim Bräunen die genannte Fläche nicht von den Strahlen erreicht wird und folglich nicht gebräunt wird, wodurch hellere Flächen nur im Bereich der Augen entstehen, die als unschön empfunden werden. Nicht berücksichtigt wird dabei die Gefahr einer dauerhaften Schädigung des Auges. Es hat daher nicht an Versuchen gefehlt, das Tragen eines Augenschutzes beim Benutzen eines Bräunungsgerätes obligatorisch zu machen.

Ein Beispiel dafür ist eine Einrichtung, die die elektrische Versorgung des Bräunungsgerätes und damit die Möglichkeit, dieses unter Einschalten der UV-Strahler-Röhren in Betrieb zu setzen, an das Tragen eines geeigneten Augenschutzes koppelte. Dies geschah in der Weise, daß für das Gerät eine elektrische oder elektronische Betriebssperre geschaltet war, die entweder ein Einschalten des Geräts verhinderte, wenn der Augenschutz nicht ordnungsgemäß angelegt war und/oder eine Betriebssperre das Gerät ausschaltete, wenn der Benutzer den Augenschutz während des Betriebs ablegte.

Solche und ähnliche Einrichtungen wurden jedoch von Benutzern, die einen Augenschutz nicht tragen wollten, umgangen und erfüllten als Schutzmaßnahme ihren Zweck nicht oder nur unvollständig.

In US 2003/011673 A1 ist eine Vorrichtung zum Schutz der Augen einer Person beim Metallschweißen oder -schneiden beschrieben, wobei die Vorrichtung ein Abschirmelement aufweist, das das visuelle Beobachten des Arbeitsprozesses erlaubt, jedoch die Augen der Person vor Lichtstrahlung schützt. Die Vorrichtung weist eine Videokamera auf, mit der der Arbeitsplatz zum Aufnehmen des Arbeitsablaufs in Form von Videosignalen erfaßbar ist, die zu einer Videoanzeigeeinheit übermittelt werden, die in dem Abschirmelement angeordnet ist.

In US-A-4 154 4513 sind Augenabdeckungen beschrieben, mit denen die Augen einer sonnenbadenden Person abgeschirmt werden können. Die Augenabdeckungen sind aus undurchsichtigem Material, während ein Rahmen, der die Augenabdeckungen abstützt, für bräunende Strahlung durchlässig ist, so daß das ganze Gesicht der Person gebräunt wird, inklusive dem Bereich um die Augen. Der Rahmen ist aus Kunststoffmaterial, das im Wesentlichen für ultraviolettes Licht transparent ist und die Augenabdeckungen sind verstellbar an den Rahmen angebaut.

In EP-A-0 612 539 ist ein Bräunungsgerät beschrieben, das aus einer Liege, einem Fluter und gegebenenfalls einem Gesichtsbräuner besteht, die einen Bräunungstunnel bilden. Aus dem Inneren der Liege, des Fluters und gegebenenfalls des Gesichtsbräuners wird UV-Strahlung in den Bräunungstunnel überführt. Entfernt von den den Bräunungstunnel begrenzenden Strahlungsaustrittsflächen ist eine zentrale Strahlungserzeugung angeordnet, die mit dem Innenraum der Liegekammer des Fluters und gegebenenfalls des Gesichtsbräuners über Lichtwellenleiter verbunden ist, die aus den den Strahlungsaustrittsflächen gegenüberliegenden Wandbereichen der Liege, des Fluters und gegebenenfalls des Gesichtsbräuners vorstehen. Jedem Lichtwellenleiterende ist eine Einrichtung zugeordnet, die eine großflächige Ausbreitung des austretenden Strahlenbündels bei gleichmäßiger Verteilung der Strahlung bewirkt.

Erfindungsgemäß wird zur Lösung des Problems vorgeschlagen, den Benutzer eines Bräunungsgeräts positiv dazu zu motivieren, den Augenschutz zu tragen. Dies geschieht in der Weise, dass man eine Einrichtung zum Schutz der Augen gegen UV-Strahlung bereitstellt, mit der optische Signale empfangen werden können, an deren Empfang der Benutzer eines UV-Bräunungsgeräts interessiert ist. Das Interesse veranlasst den Benutzer, den Augenschutz zu tragen und so - quasi nebenbei - seine Augen vor der UV-Strahlung und auch vor dem größten Teil der sichtbaren Strahlung zu schützen, die während des Bräunens von den UV-Strahler-Röhren emittiert wird.

Die Erfindung betrifft daher eine Vorrichtung zum Schutz der Augen eines Menschen oder Benutzers vor Strahlung, umfassend zumindest eine für die Strahlung wenig durchlässige Einrichtung zum Abdecken eines Auges oder beider Augen des Menschen oder Benutzers und eine Einrichtung zum Befestigen der Vorrichtung am Kopf des Menschen oder Benutzers, wobei die Vorrichtung weiter eine oder mehrere Einrichtung(en) umfaßt, die ein Empfangen optischer Signale von einem Ort ermöglichen, der von der Vorrichtung beabstandet ist, worin die Einrichtung zum Empfangen optischer Signale ein mindestens ein optisches Element umfassendes System zur optischen Übertragung sichtbarer Licht-Signale ist, worin die Einrichtung eine Öffnung in der für Strahlung wenig durchlässigen Einrichtung umfaßt, die die optischen Signale auf das mindestens eine optische Element passieren läßt, das seinerseits die optischen Signale an das Auge weiterleitet, oder worin die Einrichtung ein für sichtbares Licht durchlässiges Sichtfenster der Oberfläche in der für Strahlung wenig durchlässigen Einrichtung umfaßt, das die optischen Signale auf das mindestens eine optische Element passieren läßt, das seinerseits die optischen Signale an das Auge weiterleitet.

Die Erfindung betrifft auch ein Bräunungsgerät für kosmetische und/oder medizinische Anwendungen, das umfaßt:
(a) eine statische Liege mit einer für bräunende Strahlung transparenten Liegefläche für den Benutzer und bräunende Strahlung angebende UV-Strahler-Röhren;
(b) ein bewegliches Oberteil mit einer für bräunende Strahlung transparenten Schutzfläche und bräunende Strahlung abgebenden UV-Strahler-Röhren;
(c) wobei das Oberteil im Verhältnis zu der Liege zum Einsteigen eines Benutzers geöffnet und bei Beginn eines Bräunungsvorgangs geschlossen werden kann;
(d) vom Benutzer bedienbaren Steuergeräten zur Steuerung der Bedingungen eines Bräungsvorganges; und
(e) einer Vorrichtung zum Schutz der Augen eines Benutzers vor Strahlung, umfassend zumindest eine für die Strahlung zumindest partiell wenig durchlässige Einrichtung zum Abdecken eines Auges oder beider Augen des Benutzers und eine Einrichtung zum Befestigen der Vorrichtung am Kopf des Benutzers, wobei die Vorrichtung weiter umfaßt:
(f) eine oder mehrere Einrichtung(en), die ein Empfangen optischer Signale von einem Ort ermöglichen, der von der Vorrichtung beabstandet ist, worin die Einrichtung zum Empfangen optischer Signale ein mindestens ein optisches Element umfassendes System zur optischen Übertragung sichtbarer Licht-Signale ist, worin die Einrichtung eine Öffnung in der für Strahlung wenig durchlässigen Einrichtung umfaßt, die die optischen Signale auf das mindestens eine optische Element passieren läßt, das seinerseits die optischen Signale an das Auge weiterleitet, oder worin die Einrichtung ein für sichtbares Licht durchlässiges Sichtfenster der Oberfläche in der für Strahlung wenig durchlässigen Einrichtung umfaßt, das die optischen Signale auf das mindestens eine optische Element passieren läßt, das seinerseits die optischen Signale an das Auge weiterleitet.

Die Erfindung wird anhand der nachfolgenden Beschreibung unter Bezugnahme auf die Figuren näher erläutert. Bevorzugte Beispiele gemäß der Erfindung einschließlich solcher Ausführungsformen, wie sie in den Figuren dargestellt sind, dienen lediglich der besseren Veranschaulichung der Erfindung, ohne daß die Erfindung auf diese beschränkt ist.
Figur 1 zeigt eine schematische Darstellung einer Ausführungsform der Vorrichtung 1 gemäß der Erfindung zur Abdeckung beider Augen eines Benutzers.
Figur 2 zeigt eine schematische Darstellung einer Ausführungsform der Vorrichtung 1 gemäß der Erfindung zur Abdeckung eines Auges eines Benutzers.
Figur 3 zeigt eine schematische Darstellung einer Ausführungsform der Vorrichtung gemäß Figur 2 mit der Einrichtung zum Empfangen optischer Signale.
Figur 4 zeigt eine schematische Darstellung des Strahlengangs in einer Ausführungsform einer Vorrichtung gemäß der Erfindung.

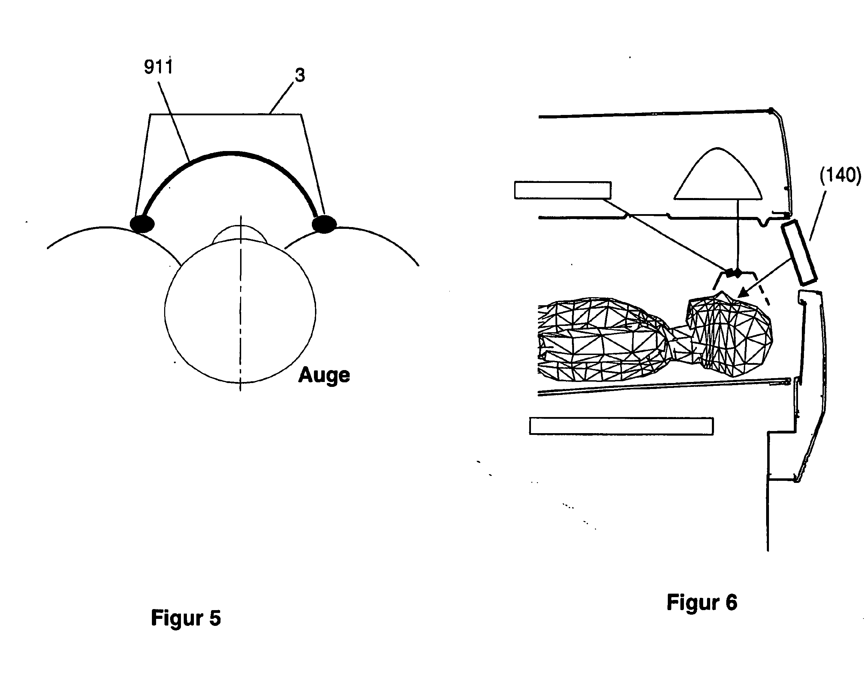

Figur 5 zeigt eine schematische Darstellung einer Ausführungsform der Vorrichtung gemäß der Erfindung mit einem LCD auf der Innenseite der Einrichtung zum Abdecken des Auges.
Figur 6 zeigt einen Teil einer Bräunungsliege gemäß der Erfindung, in der die Anordnung der Einrichtung zur Abgabe optischer Signale gezeigt ist.

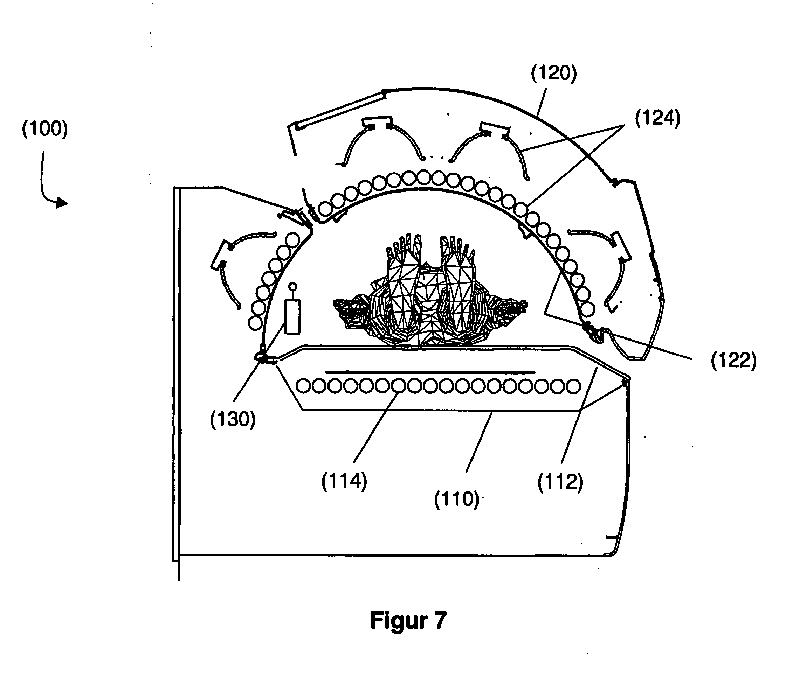

Figur 7 zeigt schematisch eine Bräunungsliege gemäß der Erfindung mit ihren verschiedenen Einrichtungen.

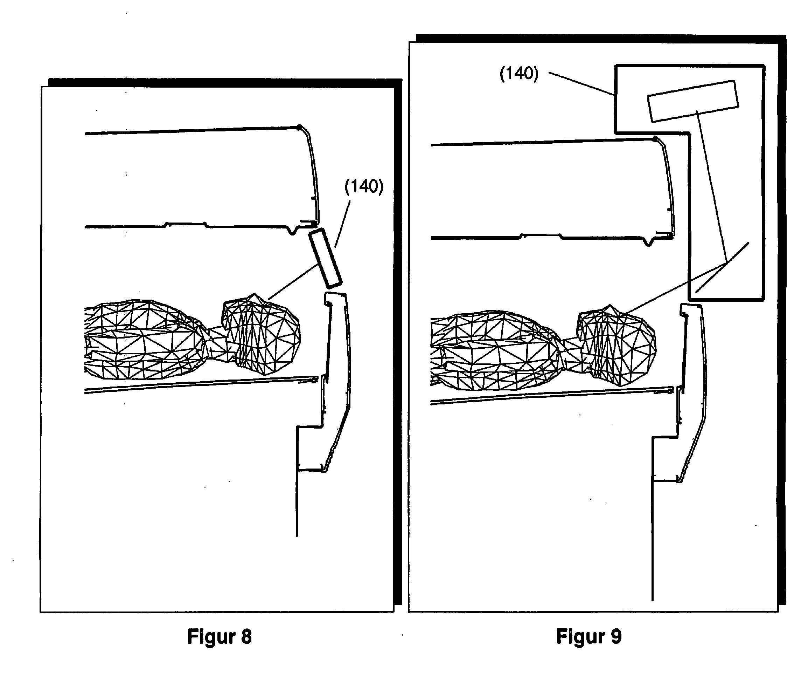

Figur 8 und Figur 9 zeigen einen Teil einer Bräunungsliege gemäß der Erfindung, in der zu Figur 6 verschiedene Anordnungen der Einrichtung zur Abgabe optischer Signale gezeigt sind.
Figur 10 zeigt schematisch eine Ausführungsform einer Bräunungsliege gemäß der Erfindung, in der Teilbereiche der von den Strahlungsquellen abgestrahlten Strahlung durch die erfindungsgemäße Vorrichtung abgeschirmt werden, während andere Bereiche der abgestrahlten Strahlung geräteseits abgeschirmt werden.

Die erfindungsgemäße Vorrichtung zum Schutz der Augen eines Menschen oder Benutzers vor Strahlung ist in ihrer allgemeinsten Ausführungsform eine Strahlenschutz-Vorrichtung, die verhindert, daß Strahlung, die aufgrund ihrer physikalischen Ausbreitung und Ausrichtung auf das Auge des Menschen auftrifft oder vor der sich der Mensch bei Exposition gegenüber dieser Strahlung nicht durch andere Mittel schützen kann, das Auge des Menschen trifft, gegebenenfalls (selbst bei geschlossenen Augenlidern) in dieses eindringt und dort schädliche Auswirkungen hat. Diese Auswirkungen können vorübergehend (und damit möglicherweise nicht auf Dauer beeinträchtigend) sein, wie sie beispielsweise dann auftreten, wenn man mit geöffneten Augen in ein helles (sichtbares) Licht - vornehmlich Licht mit einer Wellenlänge im Bereich von 400 bis 550 nm - schaut und anschließend einen hellen Fleck im Blickfeld hat, der für eine gewisse Zeit verhindert, daß das Auge/die Augen übliche optische Signale geordnet wahrnimmt/wahrnehmen. Die Auswirkungen können jedoch auch dauerhaft (insbesondere dauerhaft beeinträchtigend) sein, beispielsweise bei über längere Zeit auf das Auge/die Augen auftreffender UV-Strahlung, die den Sehnerv und/oder die Retina irreversibel schädigen kann. Gemäß einer bevorzugten, nicht jedoch beschränkenden Ausführungsform der Erfindung betrifft die Erfindung eine Vorrichtung zum Schutz des menschlichen Auges vor UV-Strahlung und/oder intensiver sichtbarer Strahlung. Weiter bevorzugt betrifft die Erfindung eine Vorrichtung zum Schutz der menschlichen Augen vor natürlicher UV-Strahlung und natürlicher sichtbarer Strahlung, wie sie beispielsweise in natürlicher Umgebung bei Sonnenschein auftritt, oder betrifft alternativ in einer Umgebung mit künstlicher UV-Strahlung und/oder künstlicher sichtbarer Strahlung auftretende Strahlung.

Ein Beispiel für natürliche UV-Strahlung und/oder natürliche sichtbare Strahlung ist die Strahlung im Gebirge oder am Meer, insbesondere dann, wenn eine Reflexion der Strahlung an dort vorhandenen Medien wie beispielsweise Schnee oder Wasser erfolgt, oder auch die Strahlung an künstlichen Gewässern. Bekannt ist, daß beispielsweise intensive Strahlung bei Schnee im Gebirge zu einer Erscheinung führt, die mit "Schneeblindheit" bezeichnet wird, wenn die Augen überstark mit dort herrschender, von der Sonneneinstrahlung stammender UV-Strahlung und/oder intensiver Strahlung im sichtbaren Bereich in Kontakt kommen.

Ein Beispiel für künstliche UV-Strahlung und/oder künstliche sichtbare Strahlung, an der die Erfindung beispielhaft erläutert wird, ohne jedoch auf diese Ausführungsform beschränkt zu sein, ist die beim Bräunen in einem Sonnenstudio auftretende künstliche UV-Strahlung und/oder künstliche sichtbare Strahlung. Im letztgenannten Fall setzt sich ein Mensch oder Benutzer mit einem Teil des Körpers, sei es nur mit dem Gesicht, sei es mit einem anderen Teil des Körpers wie mit den Extremitäten (Arme, Beine), besonders bevorzugt jedoch mit dem gesamten Körper, zum Bräunen einer für diesen Zweck geeigneten Strahlung in einem hierfür geeigneten-Bräunungsgerät aus. Die Strahlung, die von UV-Strahlungsquellen wie beispielsweise UV-Strahler-Röhren oder Nochdruck-Brennern abgestrahlt wird. enthält zumindest zu einem für die Bräunung wirksamen Teil eine UV-Komponente, wenn sie nicht sogar im wesentlichen mit dem für die Bräunung wirksamen Teil aus UV-Strahlung besteht. In den Fällen, in denen eine Bräunung in einem Bräunungsgerät erfolgt, das die Bräunung des Gesichts einschließt, wird die Strahlung regelmäßig - wie aus dem Stand der Technik bekannt - von UV-Strahler-Röhren oder Hochdruck-Brennern emittiert. Die spektrale Zusammensetzung der Strahlung wird im wesentlichen durch die in der/den Röhre(n) oder in dem Brenner enthaltenen Substanzen, regelmäßig Phosphore, bestimmt, während sich die Intensität der Strahlung apparateseitig steuern läßt, wie dem Fachmann auf diesem Gebiet hinlänglich bekannt ist und hier keiner weiteren Erläuterung bedarf. Damit läßt sich die spektrale Zusammensetzung der UV-Strahlung so steuern, daß keine die der Strahlung ausgesetzte Haut schädigenden UV-Komponenten enthalten sind.

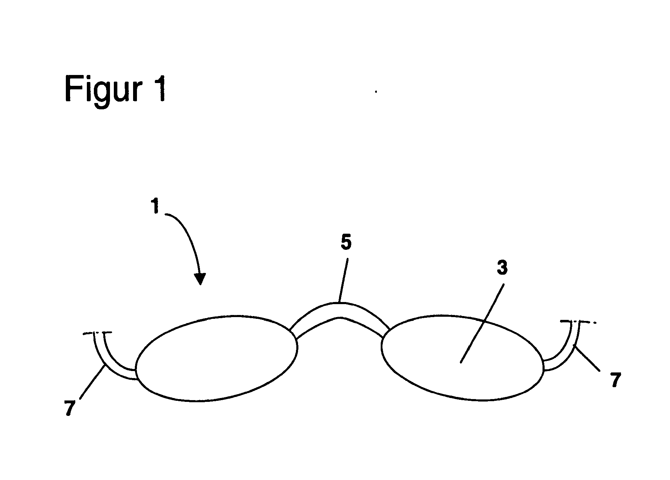

Es wird in der nachfolgenden Beschreibung auf Figur 1 Bezug genommen. Zum Schutz der Augen vor der zumindest zum Teil aus UV-Strahlung und intensiver sichtbarer Strahlung bestehenden Strahlung wird ein Auge oder werden die Augen des Menschen bzw. Benutzers mit der Vorrichtung 1 gemäß der Erfindung abgedeckt. Diese umfaßt zumindest eine für die Strahlung zumindest partiell wenig durchlässige Einrichtung 3 zum Abdecken eines Auges oder beider Augen eines Menschen oder Benutzers und eine Einrichtung 7 zum Befestigen der Vorrichtung 1 am Kopf des Menschen bzw. Benutzers, dessen Auge(n) geschützt werden soll(en). Eine Abdeckung der Auges bzw. der Augen bedeutet im Rahmen der vorliegenden Erfindung, daß die Einrichtung 3 eine solche Größe haben soll, daß zumindest der Bereich des Gesichtes des Menschen bzw. Benutzers der Vorrichtung 1, in dem sich die Augen befinden, einen rundum abdeckenden Schutz vor der Strahlung haben soll. In einer bevorzugten Ausführungsform der Erfindung schließt die Einrichtung 3 mit den Rändern der Augenhöhlen des Menschen/Benutzers lichtdicht ab, so daß kein schädliches Licht an den Rändern der Einrichtung 3 vorbei in die Augenhöhle und im Anschluß daran in das Auge eindringen kann. Dies kann in jeder dem Fachmann bekannten Weise erreicht werden, beispielsweise mit einem dicht ansitzenden Ring oder Polster.

Eine Einrichtung 3 gemäß der Erfindung kann so beschaffen sein, daß eine Einrichtung 3 Abmessungen hat, die gleichzeitig beide Augen eines Menschen bzw. Benutzers abdeckt. Dies ist eine denkbare und von der Erfindung umfaßte, erfindungsgemäß jedoch weniger bevorzugte Ausführungsform, da auch der Bereich zwischen den Augenhöhlen abgedeckt ist. In dem Fall, daß die Vorrichtung beim Bräunen in einem Bräunungsgerät verwendet wird, wird dadurch auch der Bereich zwischen den Augen abgedeckt und folglich nicht gebräunt. Dem kann in dieser (weniger bevorzugten) Ausführungsform dadurch begegnet werden, dass beispielsweise die Vorrichtung 1 im Bereich zwischen den beiden Augen eine Einrichtung aufweist, die partiell für bräunende Strahlung durchlässig ist, also beispielsweise aus einem partiell für UV-Strahlung durchlässigen Material besteht, während der die Augen abdeckende Bereich aus einem im wesentlichen für UV-Strahlung und/oder sichtbare Strahlung wenig durchlässigen oder sogar undurchlässigen Material besteht.

In anderen, erfindungsgemäß bevorzugten Ausführungsformen, von denen eine in den Figuren 1 bis 3 dargestellt ist, ist die Vorrichtung 1 so gestaltet, daß für jede Augenhöhle eine getrennte Einrichtung 3 vorgesehen wird. Dies hat den Vorteil größerer Flexibilität. Weiter tritt der vorstehend beschriebene Nachteil, beim Bräunen auch den Bereich zwischen den Augen abzudecken, mit der bevorzugten Ausführungsform nicht ein.

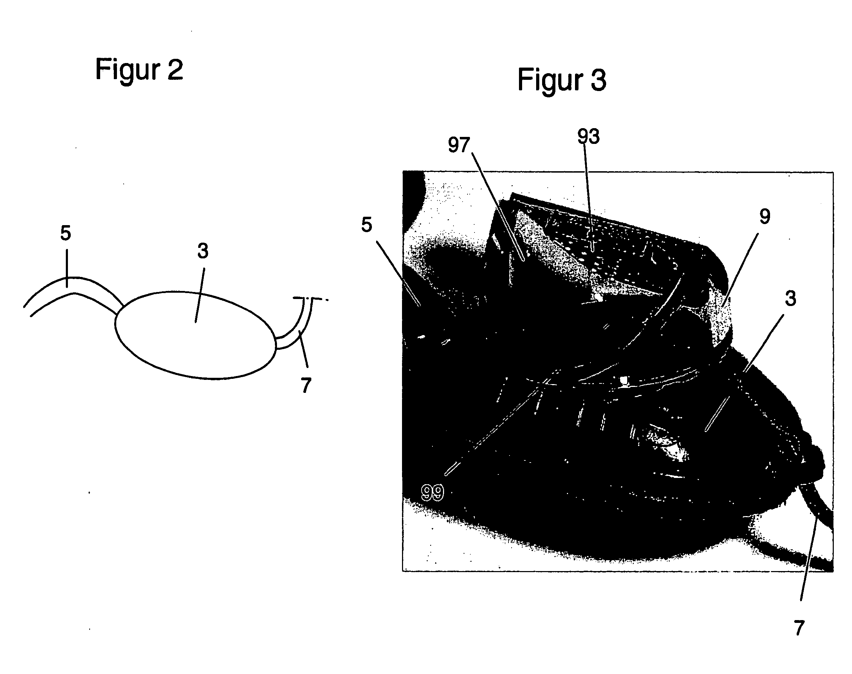

Die die Augen abdeckende(n) Einrichtung(en) 3 kann/können jede beliebige Form haben. Sie können flach (beispielsweise in Form eines Bandes) oder konturiert sein, insbesondere die Form von Raumkörpern haben (beispielsweise von runden oder ovalen Zylindern oder Halbkugeln oder Halbellipsoiden mit einer fehlenden Stirnseite), um den Augenlidern/Wimpern Raum für ein Öffnen und Schließen zu lassen. Eine andere, alternative Form ist eine flache, beispielsweise einem Kugelschalen-Segment entsprechende Form, die mittig eine Öffnung aufweist, auf die ein einseitig geöffneter Raumkörper aufgesetzt ist, der den Augenlidern/Wimpern Raum für das Öffnen bzw. Schließen läßt. Die letztgenannte Ausführungsform ist beispielsweise in Figur 3 gezeigt. Grundsätzlich sind die vorgenannten Formen Beispiele, die die Erfindung nicht beschränken; dem Fachmann sind bei der Form der Einrichtungen 3 keine Beschränkungen auferlegt, solange sie das zuverlässige Abdecken der Augen erlauben und dem Menschen/Benutzer den gewünschten Schutz gegen Strahlung bieten.

Unter dem Begriff "für Strahlung wenig durchlässig" wird im Rahmen der vorliegenden Erfindung verstanden, daß das Material der Einrichtung(en) 3 so beschaffen ist, daß die Menge an durch das Material tretender Strahlung so gering ist, daß Schädigungen der Augen nicht zu befürchten sind. Grundsätzlich gibt es für den Schutz der Augen gegen schädliche Strahlung allgemein und gegen schädliche Strahlung im UV-Bereich und/oder im sichtbaren Bereich technische Richtwerte und Normen; diesen hat das Material in jedem Fall zu genügen. Zumindest bedeutet also "für Strahlung wenig durchlässig", daß das Material oder die Materialien, aus denen die Einrichtung(en) besteht/bestehen, eine so geringe Durchlässigkeit für Strahlung allgemein, besonders bevorzugt erfindungsgemäß für UV-Strahlung und/oder sichtbare Strahlung, aufweist/auf weisen, daß den zum Schutz dienenden Richtwerten bzw. Höchstwerten Rechnung getragen wird. Von der Erfindung umfaßt sind andererseits auch Materialien für die Einrichtung(en) 3, die so beschaffen sind, daß sie gegenüber Strahlung, erfindungsgemäß besonders bevorzugt gegenüber UV-Strahlung und/oder sichtbarer Strahlung, vollständig oder im wesentlichen undurchlässig sind. Zwischenwerte zwischen den genannten Extremen sind möglich. Die Einrichtung(en) 3 kann/können aus einem Material bestehen oder kann/ können aus mehreren Materialien oder aus einem Verbund mehrerer Materialien bestehen. Diese können sich hinsichtlich ihrer Eigenschaften, insbesondere hinsichtlich ihrer jeweiligen Durchlässigkeit für Strahlung, erfindungsgemäß besonders bevorzugt für UV-Strahlung und/oder sichtbare Strahlung, unterscheiden und können sich gegebenenfalls synergistisch ergänzen.

Der Fachmann kennt Werte der Durchlässigkeit verschiedener Materialien, die für die Einrichtung(en) 3 geeignet sind, oder kann sie ohne größeren experimentellen Aufwand ermitteln.

Besonders bevorzugt ist/sind erfindungsgemäß eines oder mehrere Material(ien), dessen/deren Durchlässigkeit für UV-Strahlung mit Wellenlängen λ ≤ 400 nm, nämlich für UV-Strahlung im Bereich 400 nm ≥ λ ≥320 nm, bei höchstens 1 % liegt, bezogen auf die eingestrahlte UV-Strahlung, und dessen/deren Durchlässigkeit für UV-Strahlung mit Wellenlängen λ ≤ 320 nm, nämlich für UV-Strahlung im Bereich 320 nm ≥λ≥ 250 nm, bei höchstens 0,1 % liegt, bezogen auf die eingestrahlte UV-Strahlung. Besonders bevorzugt liegt die Durchlässigkeit des Materials/der Materialien der Einrichtung(en) 3 für UV-Strahlung bei nahezu 0 (null), bezogen auf die eingestrahlte UV-Strahlung. Die Durchlässigkeit des/der erfindungsgemäß für die Einrichtung(en) 3 verwendeten Materials/Materialien für sichtbare Strahlung (d. h. Strahlung mit Wellenlängen λ im Bereich von λ ≥400 nm, insbesondere mit Wellenlängen von 400 nm ≤ λ ≤550 nm) liegt bei höchstens 5 %, bezogen auf die eingestrahlte sichtbare Strahlung. Auch in diesem Fall der sichtbaren Strahlung liegt die Durchlässigkeit möglichst niedrig, vorzugsweise bei < 5 % der eingestrahlten sichtbaren Strahlung. In dem Fall, dass die Einrichtung(en) 3 aus einem Material besteht/bestehen, erfüllt das eine Material die oben angegebenen Spezifikationen der Durchlässigkeit für UV-Strahlung und/oder sichtbare Strahlung. Selbstverständlich müssen dabei die für den Benutzer geltenden Normen eingehalten werden können. In dem Fall, dass die Einrichtung(en) 3 aus mehreren Materialien bestehen, erfüllen die mehreren Materialien zusammen oder erfüllt eines von den mehreren Materialien allein die oben angegebenen Spezifikationen der Durchlässigkeit für UV-Strahlung und/oder sichtbare Strahlung. Diese Ausführungsformen der Erfindung umfassen auch ein Material oder mehrere Materialien, auf das/die Schichten zur Verringerung der Durchlässigkeit für Strahlung, insbesondere für UV-Strahlung und/oder sichtbare Strahlung, durch dem Fachmann an sich bekannte Verfahren aufgebracht werden, beispielsweise aufgespritzt oder aufgedampft werden. Auch die aufgebrachten Materialien können als einzelne Materialien oder als Mischungen oder Verbünde solcher Materialien aufgebracht, beispielsweise aufgespritzt oder aufgedampft, werden.

Das Material für die Einrichtung(en) 3 kann jedes an sich bekannte Material sein, solange es die oben angegebenen Spezifikationen der geringen Strahlendurchlässigkeit erfüllt. Erfindungsgemäß bevorzugt sind leicht in Massenfertigung herstellbare Materialien wie beispielsweise thermo- oder duroplastische Kunststoffe oder Glaswerkstoffe oder Mischungen oder Verbundmaterialien daraus. Denkbar ist auch, in dem Bereich der Vorrichtung 1, die das Auge bzw. die Augen des Menschen bzw. Benutzers abdeckt, ein Material oder mehrere Materialien zu verwenden, die im wesentlichen für Strahlung, insbesondere für UV-Strahlung wenig durchlässig oder undurchlässig ist/sind, während die Bereiche der Vorrichtung 1, die nicht dem Abdecken eines Auges bzw. beider Augen des Menschen bzw. Benutzers liegen, aus einem anderen Material oder mehreren anderen Materialien bestehen können, dessen/deren Durchlässigkeit für Strahlung, insbesondere UV-Strahlung, höher ist oder das/die für Strahlung, insbesondere für UV-Strahlung, im wesentlichen durchlässig ist.

Erfindungsgemäß weist die Vorrichtung 1 eine Einrichtung 7 zum Befestigen der Vorrichtung 1 am Kopf des Menschen bzw. Benutzers auf. Die Befestigungseinrichtung kann ein einfaches elastisches Band sein, das um den Kopf oder ein Ohr/beide Ohren des Menschen bzw. Benutzers gelegt wird, oder kann eine starre Einrichtung 7 sein, die um den (Hinter-) Kopf oder das Ohr/die Ohren des Menschen bzw. Benutzers geführt wird und an deren im Gesichtsbereich liegenden Enden die Einrichtung 3 bzw. (bei je einer Einrichtung 3 für jede Augenhöhle) die beiden Einrichtungen 3 befestigt sind. Dem Fachmann sind derartige Befestigungseinrichtungen in zahlreichen Erscheinungsformen und Ausführungen bekannt, so daß er sie ohne weiteres für die Vorrichtung 1 der vorliegenden Erfindung nutzbar machen kann. Bevorzugt ist die Befestigungseinrichtung 7 ein elastisches Band.

In einer weiteren bevorzugten Ausführungsform, insbesondere in dem Fall, in dem die Befestigungseinrichtung 7 ein elastisches Band ist, umfaßt die Vorrichtung 1 auch eine die beiden Einrichtungen 3 im Gesichtsbereich des Menschen bzw. Benutzers variabel oder starr verbindende Einrichtung 5. Diese dient dazu, einen zuverlässigen Sitz der gesamten Vorrichtung 1, insbesondere einen einen zuverlässigen Schutz gegen Bestrahlung bietenden Sitz der Vorrichtung 1 mit vor den Augen des Menschen bzw. Benutzers liegenden Einrichtungen 3 zu garantieren. Darüber hinaus ermöglicht eine variable Einrichtung 5 eine Anpassung der Maße der gesamten Vorrichtung 1 an die unterschiedlichen anatomischen Gegebenheiten des Benutzers. In einer besonders bevorzugten Ausführungsform ist auch die Einrichtung 5 ein elastisches oder starres Band, mit besonderem Vorteil ein elastisches Band.

Erfindungsgemäß umfasst die erfindungsgemäße Vorrichtung 1 eine oder mehrere Einrichtung(en) 9, die ein Empfangen optischer Signale von einem Ort ermöglicht/ ermöglichen, der von der Vorrichtung 1 beabstandet ist. Darunter wird im Rahmen der vorliegenden Erfindung verstanden, dass von einem Ort (denkbarerweise auch von mehreren Orten), der/die sich in einer mehr oder weniger großen Entfernung von der Vorrichtung 1 befindet, optische Signale an die Vorrichtung gesendet oder geschickt werden können, die mittels der Vorrichtung 1 empfangen und dem die Vorrichtung 1 tragenden Menschen bzw. Benutzer zur Ansicht gebracht werden können. Die Entfernung kann beliebig groß in dem Fall sein, in dem der Empfang optischer Signale drahtlos erfolgt. Denkbar ist für diesen Fall beispielsweise eine Sender-/Empfänger-Anordnung, bei der der Sender an beliebiger Stelle steht und die Vorrichtung 1 über einen geeigneten Empfänger verfügt. Alternativ dazu kann der Empfänger in der Nähe der Vorrichtung verfügbar gehalten werden (beispielsweise ein statischer Empfänger in direkter Umgebung der Vorrichtung 1 oder ein mobiler [z. B. tragbarer] Empfänger in direkter Umgebung der Vorrichtung 1), und in einer bevorzugten Ausführungsform kann ein solcher Empfänger mit einer Kabelverbindung oder einer ähnlichen, dem Fachmann an sich bekannten Weise mit der Vorrichtung 1 zur Übertragung der optischen Signale oder zur Übertragung elektrischer oder elektronischer Signale und deren Umwandlung in optische Signale verbunden sein, die dem Auge/den Augen des Menschen bzw. Benutzers der Vorrichtung sichtbar gemacht werden.

Von der Erfindung ebenfalls umfasst ist eine bevorzugte Ausführungsform, gemäß der die optischen Signale dadurch sichtbar gemacht werden, dass die Einrichtung(en) 3 der Vorrichtung 1 auf ihrer Innenseite, die beim Tragen der Vorrichtung 1 dem Auge/den Augen des Menschen bzw. Benutzers zugewandt ist/sind, mindestens ein Display, beispielsweise ein LCD, das ein Mikrodisplay sein kann, oder mindestens eine Schicht oder mindestens einen Film aufweisen, der/die die Wiedergabe optischer Signale in einer für das geschützte Auge bzw. die geschützten Augen wahrnehmbaren Form, insbesondere in sichtbarer Form, erlaubt. Ganz besonders bevorzugt ist ein LCD 911. Die Einrichtung(en), beispielsweise Display(s), Schicht(en), Film(e), auf der Innenseite der jeweiligen Einrichtung 3 der Vorrichtung 1 wird/werden von einem direkt an der Vorrichtung angebrachten Empfänger oder über eine Kabelverbindung angesteuert, und die empfangenen Signale werden in an sich für den Fachmann bekannter Weise in optische Signale umgesetzt, die auf dem Film 911 sichtbar werden und damit für das Auge/die Augen wahrnehmbar sind. Auf diesem Weg ist die Übertragung von Signalen in optischer, für das Auge wahrnehmbarer Weise von einem Ort, der von der Vorrichtung 1 mehr oder weniger weit beabstandet ist, oder sogar von mehreren derartigen Orten zu der Vorrichtung 1 möglich. Der Abstand kann wenige Zentimeter bis Hunderte von Kilometern betragen, ohne dass die Erfindung hierdurch beschränkt wird.

Gemäß weiteren bevorzugten Ausführungsformen der Erfindung werden die optischen Signale dadurch empfangen und/oder sichtbar gemacht, dass auf der Innenseite der Einrichtung(en) 3 eine Vielzahl kleiner (gegebenenfalls beweglicher) Spiegel befindet, die optische Mikroarrays erzeugen, die von dem vor schädlicher Strahlung geschützten Auge als Bilder wahrgenommen und so betrachtet werden können.

Der wesentliche Vorteil dieser vorgenannten Ausführungsformen ist, dass der Empfang der optischen Signale vom Sender im wesentlichen unabhängig erfolgt und damit eine vollständige Mobilität des Menschen/Benutzers der Vorrichtung 1 garantiert ist. Weiter ist die Art der empfangenen optischen Signale nicht auf spezielle Sender beschränkt, sondern lässt die volle Bandbreite des Empfangs im wesentlichen überall zu. Beispielsweise (ohne die Erfindung hierdurch zu beschränken) kann ein Bergwanderer beim Wandern in den Bergen seine Augen vor natürlicher UV-Strahlung und sichtbarer Strahlung mit einer als Brille ausgestalteten Vorrichtung 1 gemäß der Erfindung schützen und kann gleichzeitig optische Signale von weit entfernten Orten empfangen, beispielsweise Unterhaltungs-Signale oder Sach-Signale, aber auch Informationen über sich ändernde Wetterverhältnisse und/oder Weg-Informationen in Form einer Wanderkarte oder Informationen über Flora und Faune der Gegend, in der sich der Benutzer gerade befindet, o. ä.. In den letztgenannten Ausführungsformen, in denen die Informationen sachorientiert sind, kann z.B. eine Kopplung mit einem an sich bekannten GPS-System geschaffen werden, um für die Vorrichtung erkennbar zu machen, wo sich der Benutzer gerade befindet, um die Sachinformationen an den Ort anzupassen, sobald dies erforderlich ist.

Eine andere, erfindungsgemäß besonders bevorzugte Ausführungsform der Vorrichtung 1 dient - wie bereits oben ausgeführt - zum Schutz der Augen des Menschen bzw. Benutzers vor künstlicher Strahlung, sei es künstlicher UV-Strahlung und/oder künstlicher sichtbarer Strahlung. In weiter bevorzugten Ausführungsformen stammt solche UV-Strahlung und/oder sichtbare Strahlung von solche Strahlung erzeugenden Geräten, beispielsweise Schweißgeräten, einen Lichtbogen erzeugenden Geräten oder - gemäß besonders bevorzugten Ausführungsformen der Erfindung - von Geräten zum Bräunen der Haut eines Menschen bzw. Benutzers. Solche Bräunungsgeräte sind - wie dem Fachmann bekannt ist - mit Strahlungsquellen, beispielsweise UV-Strahlungsquellen ausgestattet, die beispielsweise Strahlung im UV-Bereich und/oder sichtbaren Bereich abgeben. Beispiele solcher Strahlungsquellen sind Röhren oder Hochdruck-Brenner. Die von solchen Strahlungsquellen im Betrieb abgestrahlte Strahlung bräunt die Haut in sanfter und ungefährlicher Weise, wenn sie richtig eingesetzt wird, ist jedoch für das ungeschützte Auge zumindest unangenehm (sichtbare Strahlung) oder gar schädlich (UV-Strahlung).

Infolge dieser Gefahr sollte der Mensch bzw. Benutzer beim Bräunen einen Schutz für die Augen vor UV-Strahlung, passenderweise auch vor der intensiven Strahlung im sichtbaren Bereich tragen. In ganz besonders bevorzugten Ausführungsformen der Erfindung umfasst daher die Vorrichtung 1 auch eine oder mehrere Einrichtung(en), die eine Kontrolle darüber ermöglichen, ob der Mensch bzw. Benutzer eines Bräunungsgeräts eine Vorrichtung 1 zum Schutz der Augen vor UV-Strahlung und/oder Strahlung im sichtbaren Bereich ordnungsgemäß trägt, d. h. also so trägt, dass das angestrebte Ziel, nämlich der Schutz der Augen vor der UV-Strahlung und/oder der sichtbaren Strahlung, erreicht wird. Mit besonderem Vorteil wird dies erfindungsgemäß dadurch erreicht, dass zwischen dem Bräunungsgerät und der erfindungsgemäßen Vorrichtung 1 eine Kontroll-Einrichtung geschaltet ist, die gewählt ist aus elektrischer Kontroll-Einrichtung, elektronischer Kontroll-Einrichtung, funkgesteuerter Kontroll-Einrichtung und einer Kombination einer oder mehrerer derartiger Einrichtungen. Grundsätzlich sind solche Einrichtungen dem einschlägigen Fachmann bekannt und bedürfen daher an dieser Stelle keiner detaillierten Erklärung. Solche Kontroll-Einrichtungen funktionieren üblicherweise so, dass dem Bräunungsgerät auf einem der genannten Wege "gemeldet" wird, ob der Mensch bzw. Benutzer die Vorrichtung 1 ordnungsgemäß trägt, d. h. so, dass seine Augen vor UV-Strahlung und/oder sichtbarer Strahlung entsprechend den üblichen Vorgaben, beispielsweise gemäß den oben im Zusammenhang mit den für die Einrichtungen 3 dargelegten Vorgaben, der Durchlässigkeit für UV-Strahlung und/oder sichtbare Strahlung geschützt sind. Dies wird auch weiter unten noch im Detail erläutert. Ist der vorgegebene Schutz der Augen nicht gewährleistet, schaltet das Bräunungsgerät nicht ein, d. h. ein Bräunungsvorgang kann nicht beginnen. Wenn der Schutz nicht mehr fortbesteht, beispielsweise weil der Mensch/ Benutzer die Vorrichtung ablegt, schaltet sich das Bräunungsgerät ab, d. h. der Bräunungsvorgang wird unterbrochen.

Auch im Rahmen der Benutzung der erfindungsgemäßen Vorrichtung 1 beim Bräunen in einem Bräunungsgerät kann die Übertragung optischer Signale an die Vorrichtung 1 wie im vorangehend dargelegten Fall erfolgen: Optische Signale oder in optische Signale umwandelbare elektrische oder elektronische Signale können von einem Ort, der von der Vorrichtung 1 mehr oder weniger weit beabstandet ist, an die Vorrichtung 1 über-, tragen werden und werden in dieser - beispielsweise über einen Film auf der Innenseite der Einrichtung(en) 3, d. h. auf der dem Auge/den Augen zugewandten Seite, für das Auge/die Augen des Menschen bzw. Benutzers sichtbar gemacht.

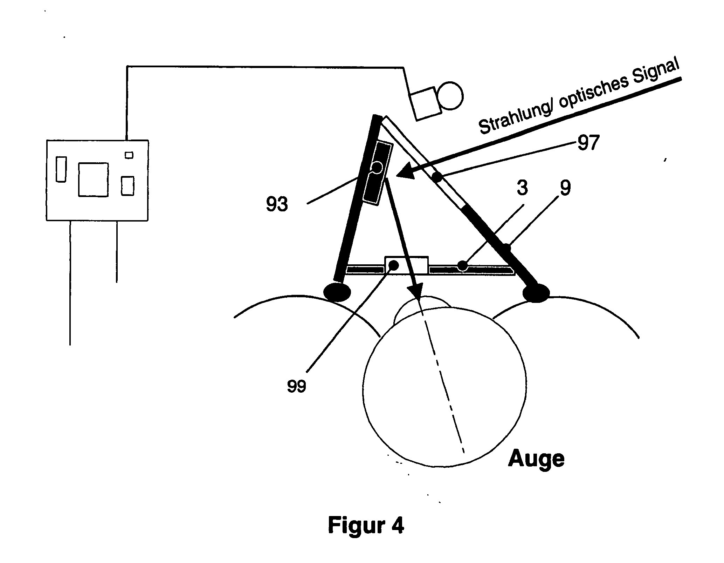

Erfindungsgemäß weist, wie es schematisch in Figur 4 gezeigt ist, die Einrichtung 9 zum Empfangen optischer Signale ein mindestens ein optisches Element 93 umfassendes System zur optischen Übertragung sichtbarer Licht-Signale auf. Darunter wird erfindungsgemäß verstanden, ohne die Erfindung hierauf zu beschränken, dass von einem Ort, der von der Vorrichtung 1 (und auch von dem Bräunungsgerät, in dem die Vorrichtung 1 benutzt wird) beabstandet eine direkt optische Signale abgebende Einrichtung angeordnet ist. Die optische Signale abgebende Einrichtung kann jede dem Fachmann mann bekannte oder aufgrund seines Fachwissens einfallende Einrichtung zur Abgabe optischer Signale sein. Beispielhaft werden als bevorzugte Ausführungsformen der Erfindung Displays, Bildschirme (Fernsehen, Computer, Handheld, Handys) als Einrichtung zur Abgabe optischer Signale genannt, ohne die Erfindung hierauf zu beschränken. Eine solche optische Signale abgebende Einrichtung ist in dem bevorzugten Fall der Verwendung der Vorrichtung 1 im Zusammenhang mit dem Bräunen in einem Bräunungsgerät üblicherweise außerhalb des Aufenthaltsbereichs des Menschen bzw. Benutzers des Bräunungsgeräts angeordnet. Dies muß jedoch nicht notwendigerweise so sein. Bevorzugte Anordnungen sind eine Anordnung der Einrichtung zur Abgabe optischer Signale am Kopfende des Bräunungsgeräts oder seitlich des Bräunungsgeräts, bevorzugterweise ebenfalls in der Höhe des Kopfes des Menschen bzw. Benutzers.

Die optischen Signale sind in einer weiter bevorzugten Ausführungsform Licht-Signale im sichtbaren Bereich. Die abgegebenen Signale werden in der von der Vorrichtung 1 umfaßten Einrichtung 9 zum Empfangen optischer Signale, besonders bevorzugt von dem mindestens ein optisches Element 93 umfassenden System zur optischen Übertragung sichtbarer Licht-Signale, empfangen und so verarbeitet, dass die empfangenen Signale für das Auge sichtbar werden.

In weiter gemäß der Erfindung bevorzugten Ausführungsformen ist das optische Element 93 des Systems zur optischen Übertragung sichtbarer Licht-Signale gewählt aus einem oder mehreren Spiegel(n), einem Prisma oder mehreren Prismen, und Kombinationen der genannten optischen Elemente. Der Spiegel kann ein üblicher Spiegel sein, der keine optische Zusatzwirkung (über das Spiegeln hinaus) erzielt, oder kann ein Standard-Spiegel mit Spiegelwirkung auf der Frontfläche sein, die gegebenenfalls Zusatzwirkungen wie Vergrößern und/oder Anpassen an optische Fehlsichtigkeit bewirken kann. Wenn in einer alternativen Ausführungsform ein Prisma verwendet wird, kann dieses Prisma ein Reflexionsprisma mit oder ohne zusätzliche optische Wirkungen (wie gegebenenfalls Vergrößern und/oder Anpassung an Fehlsichtigkeit) sein. Es können mehrere Spiegel oder mehrere Prismen oder eine Kombination aus Prisma/Prismen und Spiegel/Spiegeln verwendet werden.

In der Praxis wird/werden also bevorzugt das/die optische(n) Signal(e) von der das/die Signal(e) abgebenden Einrichtung abgegeben und direkt (d. h. ohne Umwege) dem mindestens ein optisches Element 93 umfassenden System zugeleitet, bevorzugt der Oberfläche eines Spiegels oder mehrerer Spiegel oder - ebenfalls bevorzugt - der Oberfläche eines Prismas oder mehrerer Prismen oder einer Kombination daraus zugeleitet. Von dort wird das Bild auf das Auge bzw. die Augen des Menschen bzw. Benutzers der Vorrichtung 1 gespiegelt bzw. reflektiert bzw. gebrochen. Denkbar - wenn auch erfindungsgemäß weniger bevorzugt - ist, dass der Weg der optischen Signale über mehrere Spiegel, Prismen oder eine Kombination derartiger Elemente führt; bevorzugt ist jedoch ein Spiegel oder Prisma wegen des einfacheren Aufbaus und der originalgetreueren Wiedergabe der von der Quelle der sichtbaren Signale abgegebenen Licht-Signale. Erfindungsgemäß besonders bevorzugt ist eine Ausführungsform der Vorrichtung 1, bei der der Winkel α, mit dem das sichtbare Licht, von der diese Signale abgebenden Einrichtung, in die Einrichtung 1 eintritt bevorzugt im Bereich von 5 ° bis 75 ° zur Horizontalen liegt.

Erfindungsgemäß ist die Erfindung eine Vorrichtung 1, in der die Einrichtung 9 zum Empfangen optischer Signale eine Öffnung in der für Strahlung wenig durchlässigen Einrichtung 9 umfaßt, die die optischen Signale auf das mindestens eine optische Element 93 passieren lässt, das seinerseits - wie oben im Detail beschrieben - die optischen Signale an das Auge bzw. die Augen des Menschen bzw. des Benutzers weiterleitet. Alternativ dazu ist es ebenfalls erfindungsgemäß , wenn die Einrichtung 9 ein für sichtbares Licht durchlässiges Sichtfenster 97 der Oberfläche in der für Strahlung wenig durchlässigen Einrichtung 9 umfaßt, das die optischen Signale auf das mindestens eine optische Element 93 passieren lässt, das seinerseits - wie oben im Detail beschrieben - die optischen Signale an das Auge bzw. die Augen des Menschen bzw. Benutzers weiterleitet. Hiermit ist unabhängig voneinander gewährleistet, dass durch die Eigenschaft der Einrichtung(en) 9, wenig für Strahlung (bevorzugterweise einschließlich sichtbarer Strahlung) durchlässig zu sein, die eingestrahlten, zur Übermittlung an das Auge bzw. die Augen des Menschen bzw. Benutzers bestimmten optischen Signale an das Auge geleitet werden können, ohne dass dadurch die UV-Strahlung bzw. sichtbare Strahlung der UV-Strahler-Röhren oder ein Teil davon auf bzw. in das Auge bzw. die Augen gelangt.

In dem Fall, in dem die Einrichtung 9 ein für sichtbares Licht durchlässiges Sichtfenster 97 aufweist, ist dieses Sichtfenster ein Fenster aus einem beliebigen, für diesen Zweck geeigneten und für den Fachmann leicht aus einer bekannten Gruppe von Materialien auswählbaren Material. Beispiele - ohne die Erfindung hierauf zu beschränken - sind Glas und verschiedene Kunststoffe. Weiter bevorzugt ist das Sichtfenster in das Material der Einrichtung 9 nachträglich passgenau eingesetzt.

Mit den vorgehend beschriebenen bevorzugten Ausführungsformen der Erfindung wird also zwischen der optische Signale abgebenden Vorrichtung (beispielsweise einem Display eines Computers) und dem Auge bzw. den Augen des Menschen bzw. des Benutzers ein nur eine Zwischenstufe umfassender Strahlungsweg geschaffen, ohne dass optische Signale verloren gehen, beispielsweise durch das Material der Einrichtung(en) 9, das wenig für UV-Strahlung und/oder sichtbare Strahlung durchlässig sein soll, um das Auge bzw. die Augen des Menschen bzw. des Benutzers des Bräunungsgeräts zu schützen. Bevorzugt - und im Ergebnis auch erwünscht - ist das mindestens eine optische Element (beispielsweise ein Spiegel) im Verhältnis zum Auge des Menschen bzw. Benutzers der Vorrichtung 1 so im Lichtweg der optischen Signale angeordnet, dass nur die (gewünschten) optischen Signale das Auge bzw. die Augen erreichen, nicht jedoch UV-Strahlung oder sichtbare Strahlung, die von den UV-Strahler-Röhren stammen. Der Lichtweg der optischen Signale wird also so gewählt, dass er in keinem Fall mit dem Lichtweg der UV-Strahlung und/oder sichtbaren Strahlung zusammenfällt, die von den UV-Strahler-Röhren-stammen. Dadurch wird zuverlässig vermieden, dass das Auge/die Augen des Menschen bzw. Benutzers mit UV-Strahlung und/oder sichtbarer Strahlung von den UV-Strahler-Röhren in Kontakt kommt. Der Fachmann kann diese Wahl des Lichtwegs unter Vermeidung des Zutritts von UV-Strahlung und/oder sichtbarer Strahlung von den UV-Strahler-Röhren durch wenige einfache Versuche anhand der Grundgesetze der Optik bestimmen, und dies muß daher an dieser Stelle nicht im Detail erläutert werden.

Ganz besonders bevorzugt ist erfindungsgemäß eine Ausführungsform der Vorrichtung 1, bei der UV-Strahlung und/oder sichtbare Strahlung, die von UV-Strahler-Röhren eines Bräunungsgeräts stammen, durch einen in die Einrichtung 3 eingebauten Shutter 99 abgeschirmt werden. Eine derartige Ausführungsform hat den Vorteil, dass der Shutter 99 im geschlossenen Zustand eine zuverlässige Abschirmung der UV-Strahlung und/oder sichtbaren Strahlung von den UV-Strahler-Röhren garantiert und so verhindert, dass solche Strahlung das Auge bzw. die Augen des Menschen bzw. Benutzers erreicht, ohne dass der Seh-Eindruck gestört wird, der von den sichtbaren LichtSignalen stammt und dessen Durchtritt zum Auge bevorzugt ist. Dies ist insbesondere bei Berücksichtigung der Tatsache von Wichtigkeit, dass der Lichtweg zwischen der Einrichtung zur Aussendung optischer Signale über die Öffnung oder das Sichtfenster 97 in der Oberfläche der Einrichtung 9 und über das mindestens eine optische Element 93 zum Auge oder zu den Augen des Menschen bzw. Benutzers des Bräunungsgeräts nur dann "geschlossen" ist (also das Licht erfolgreich auf das Auge gelangt), wenn ganz bestimmte Winkel eingehalten werden. Der Winkel des mindestens einen optischen Elements ist bevorzugt (wenn auch nicht notwendigerweise) ein für die Vorrichtung 1 festgelegter (und damit nicht immer frei wählbarer) Parameter. Da die Haltung des Kopfes des Menschen bzw. Benutzers, der die Vorrichtung 1 insgesamt trägt, jedoch variabel ist, kann beim Bewegen des Kopfes der Lichtweg unterbrochen werden. Im Ergebnis könnte in einem solchen Fall UV-Strahlung und/oder sichtbare Strahlung durch die Öffnung oder das lichtdurchlässige Sichtfenster 97 in der Einrichtung 9 und damit auf das mindestens eine optische Element 93 und letztlich in das Auge bzw. die Augen des Menschen bzw. Benutzers dringen. Dies ist jedoch unerwünscht und wird durch den Shutter 99 erfolgreich verhindert.

Der Shutter 99 kann jeder beliebige Shutter sein, den Fachleute in diesem technischen Bereich für die oben geschilderten Zwecke kennen. Besonders bevorzugt sind erfindungsgemäß mechanische Shutter und optische Shutter, die beispielsweise übliche, an sich bekannte LCD-Shutter sein können. Optische Shutter 99 sind besonders bevorzugt.

Weiter ist eine erfindungsgemäße Ausführungsform der Vorrichtung 1 ganz besonders bevorzugt, gemäß der der Shutter 99 steuerbar ist und insbesondere in Abhängigkeit von Daten steuerbar ist, die von einem in die Einrichtung 9 eingebauten Sensor ermittelt werden. Der Sensor kann ein beliebiger Sensor sein, ohne die Erfindung zu beschränken, beispielsweise ein mechanischer Sensor, ein elektro-optischer Sensor, eine Photodiode, gegebenenfalls mit einem in einen bestimmten Wellenlängenbereich wirksamen Filter (beispielsweise einem Bandfilter im Bereich von 400 bis 550 nm) auf eine bestimmte Wellenlänge gerichteten Kantenfilter (beispielsweise ein Kantenfilter für die Wellenlänge 500 nm) oder ein anderer beliebiger Sensor.

Der Sensor ermittelt Daten, und die ermittelten Daten werden an eine Steuerung, die in der bevorzugten Ausfiihrungsform ebenfalls umfasst ist, weitergeleitet. Die Steuerung steuert den Shutter 99 in Abhängigkeit von den ermittelten Daten.

Grundsätzlich kann die Steuerung derart erfolgen, dass in einer weiter bevorzugten Ausführungsform der Erfindung der Sensor die Lage der Vorrichtung (1) in Bezug auf die Lichtquelle (z. B. mechanischer Sensor) und/oder das in die Einrichtung 9 einfallende Licht einer bestimmten Wellenlänge, beispielsweise das Licht aus den UV-Strahler-Röhren, oder eine andere physikalische Größe, beispielsweise eine dem in die Einrichtung 9 einfallenden Licht äquivalente physikalische Größe, ermittelt (elektro-optischer Sensor). Die ermittelten Daten werden an die Steuerung geleitet. In Abhängigkeit von der vom Sensor ermittelten Größe und im Anschluß an einen Vergleich der ermittelten Größe mit vorgegebenen Werten derselben Größe steuert die Steuerung den Shutter 99 an und schließt diesen oder öffnet diesen, je nach dem Ergebnis des Datenvergleichs. Das Schließen oder Öffnen kann ein vollständiges Schließen oder Öffnen oder ein partielles Schließen oder Öffnen sein. Das Schließen oder Öffnen kann in Abhängigkeit von den ermittelten Daten stufenlos oder in Stufen erfolgen. Dies hat zum Ergebnis, dass bei einer Steuerung des Shutters 99 auf diese Weise auf das mit der Vorrichtung 1 geschützte Auge des Menschen bzw. Benutzers des Bräunungsgeräts eine Menge an Strahlung (besonders bevorzugt eine Menge an UV-Strahlung und/oder sichtbarer Strahlung, ganz besonders solcher Strahlung aus den UV-Strahler-Röhren) fällt, die höchstens gleich der oder noch mehr bevorzugt kleiner als die höchstzulässige Menge an Strahlung ist. Noch weiter bevorzugt ist, dass keine schädliche Strahlung auf das Auge bzw. die Augen des Menschen bzw. des Benutzers des Bräunungsgeräts fällt, wenn die Steuerung den Shutter 99 schließt, beispielsweise bei einer Neigung des Kopfes des Menschen bzw. Benutzers, die nach den mechanischen Daten einen Strahlungseinfall auf das Auge zur Folge haben müsste, oder bei Erfassen von UV-Strahlung und/oder sichtbarer Strahlung aus den UV-Strahler-Röhren in der Einrichtung 9 durch den Sensor und Weitergabe entsprechender Daten vom Sensor an die Steuerung des Shutters 99.

Die für die vorstehend beschriebene, Shutter 99, Sensor und Steuerung umfassende Ausführungsform erforderliche Elektronik, einschließlich Software für deren Auswertung, ist dem Fachmann als solche bekannt, und er kann diese Komponenten anhand seiner Fachkenntnis für die jeweiligen Zwecke auswählen und mit der geschilderten Hardware verknüpfen. Dies muß an dieser Stelle nicht im Detail erläutert werden.

Eine weitere bevorzugte Ausführungsform der erfindungsgemäßen Vorrichtung 1 umfasst den Fall, dass - im Gegensatz zur oben beschriebenen Ausführungsform der Vorrichtung 1, bei der beide Einrichtungen 3, die die Augen des Menschen bzw. Benutzers abdecken, eine oder mehrere Einrichtung(en) 9 umfassen, die ein Empfangen optischer Signale von einem Ort ermöglichen der von der Vorrichtung 1 beabstandet ist - nur eine der beiden Einrichtungen 3, die die Augen des Menschen bzw. Benutzers abdecken, eine oder mehrere Einrichtungen 9 umfaßt, die ein Empfangen optischer Signale von einem Ort ermöglichen der von der Vorrichtung 1 beabstandet ist (monokulare Ausführungsform), während die andere Einrichtung 3 nur das Auge des Menschen bzw. Benutzers gegen schädliche Strahlung wie UV-Strahlung und/oder sichtbare Strahlung abdeckt, ohne einen Empfang optischer Signale zu ermöglichen. Diese Ausführungsform, die entweder so ausgeführt werden kann, dass das linke Auge die empfangenen Signale über das entsprechende mindestens eine optische Element empfängt oder alternativ das rechte Auge, während das jeweils andere Auge des Menschen bzw. Benutzers keine Signale empfängt, hat erfindungsgemäß den Vorteil, dass die Vorrichtung 1 und insbesondere das optische Element 93 keinen exakten optischen Abgleich zwischen zwei Augen eines Menschen bzw. Benutzers benötigt, wie er bei einer Lösung erforderlich ist, die optische Elemente 93 für beide Augen des Menschen bzw. Benutzers umfasst. Die "monokulare" Lösung kann also insbesondere vorteilhaft sein, wenn die Entfernung zwischen sichtbare Lichtsignale abgebender Einrichtung und Vorrichtung 1 gering ist oder die beiden Augen des Menschen bzw. Benutzers für den Empfang optischer Signale unterschiedlich gut befähigt sind (unterschiedliches Sehvermögen der Augen). Demgegenüber ist ein Vorteil einer binokularen Lösung, wie sie oben beschrieben wurde, dass Sehschwächen des einen Auges des Menschen bzw. Benutzers durch ein sehstärkeres zweites Auge ausgeglichen werden können, was für den Benutzer ein besseres Erfassen der optischen Signale bedeutet.

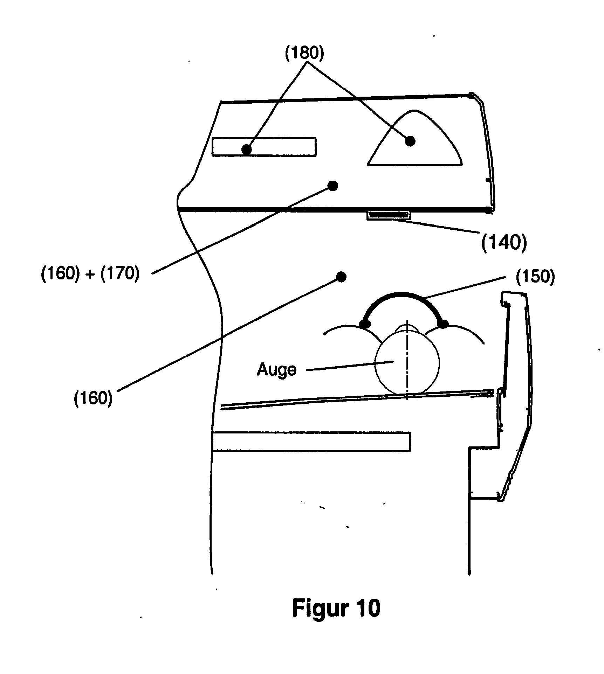

In einer weiteren bevorzugten, in Figur 10 schematisch dargestellten Ausführungsform erfolgt eine Abschirmung des Auges/der Augen eines Menschen bzw. Benutzers gegenüber schädlicher Strahlung, beispielsweise UV-Strahlung 160 und/oder sichtbarer Strahlung 170, nicht allein durch die erfindungsgemäße Vorrichtung 1. Vielmehr wird die von einer oder mehreren Strahlungsquelle(n) 180 abgestrahlte Strahlung entsprechend den Strahlungsanteilen unterschiedlicher Wellenlängen zum einen durch das Material der das Auge abdeckenden Einrichtung(en) und zum anderen durch eine oder mehrere in der Nähe der Strahlungsquellen angebrachte Einrichtung(en) abgeschirmt. In einer erfindungsgemäß besonders bevorzugten Ausführungsform kann beispielsweise eine Abschirmung gegen sichtbare Strahlung durch eine Filtereinrichtung 150 erfolgen, die in der Nähe der Strahlungsquelle(n) angeordnet ist, beispielsweise in einem Bräunungsgerät auf der Liegefläche bzw. der Schutzfläche aufgebracht oder an dieser angeordnet ist, die sich zwischen den Strahlungsquellen und dem Menschen bzw. Benutzer befindet. Die (in diesem Fall für das Bräunen gewünschte) UV-Strahlungskomponente dringt durch diese Filtereinrichtung 150 hindurch und kann auf den Körper auftreffen. Im Bereich der Augen des Menschen bzw. Benutzers wird die UV-Strahlung jedoch durch die für UV-Strahlung wenig durchlässige oder gar undurchlässige Einrichtung 3 abgeschirmt. So wird ein zuverlässiger Schutz der Augen gegen UV-Strahlung gewährleistet. Selbstverständlich kann diese Ausführungsform auch einen in die Einrichtung 3 eingebauten Shutter sowie die oben beschriebenen Sensor- und Steuereinrichtungen umfassen.

Gemäß einer weiteren, ebenfalls bevorzugten Ausführungsform der Erfindung umfasst die Vorrichtung 1 Einrichtungen, die ein Belüften der Einrichtung 3 ermöglichen oder ein Beschlagen der Einrichtung 3 durch Feuchte, Schweiß o. ä. verhindern. Lüftungseinrichtungen können geeignete Schlitze (vorzugsweise zur Vermeidung von Strahlungseintritt versetzt) sein, die einen Luftaustausch mit der Umgebung erlauben, jedoch auch andere mechanische Einrichtungen, die einer Belüftung dienen. Alternativ dazu oder zusätzlich kann eine Fläche oder können mehrere Flächen der Einrichtung(en) 3, insbesondere diejenigen, die im Strahlengang der optischen Signale liegen, aus einem ein Beschlagen verhindernden Material hergestellt sein oder mit einem solchen Material beaufschlagt (beispielsweise besprüht oder bedampft) sein.

Die optischen Signale, die mittels des mindestens einen optischen Elements 93 der Einrichtung 9 und letztlich dem Auge/den Augen des Menschen bzw. Benutzers zugeführt werden können, können beliebige optische Signale sein. In besonders bevorzugten Ausführungsformen sind die optischen Signale gewählt aus Fernsehfilm-Signalen, Kinofilm-Signalen, Videofilm-Signalen, CD-Film-Signalen, DVD-Film-Signalen, Internet-Signalen, Musicclip-Signalen, Handy-Bildschirm-Signalen, Videospiel-Signalen, Spiele-Signalen und Spielkonsole-Signalen, wobei dies jedoch die Erfindung nicht beschränkt und auch andere optische Signale gewählt werden können. Eine Steuerung der Signale (beispielsweise die Wahl eines Programms, der Wechsel von einem Programm zum anderen, interaktives Spielen, usw.) können in an sich bekannter Weise gesteuert werden, beispielsweise mit einer Kombination aus mechanischem und elektrischem Pulsgeber, einem sogenannten Joystick oder einer ähnlichen, an sich bekannten Einrichtung. Eine solche Einrichtung kann gegebenenfalls in dem Fachmann bekannter Weise auch mit Sicherheitseinrichtungen, beispielsweise mit einem Knopf zum Einschalten von Einrichtungen im Zusammenhang mit dem Bräunungsvorgang, gekoppelt oder räumlich verbunden werden. Diese Einrichtungen beschränken jedoch die Erfindung nicht.

Weiter betrifft die vorliegende Erfindung auch ein Bräunungsgerät 100. Das erfindungsgemäße Bräunungsgerät 100 umfaßt folgende Komponenten:
(a) eine statische Liege 110 mit einer für bräunende Strahlung transparenten Liegefläche 112 für den Benutzer und bräunende Strahlung angebende UV-Strahler-Röhren 114;
(b) ein bewegliches Oberteil 120 mit einer für bräunende Strahlung transparenten Schutzfläche 122 und bräunende Strahlung abgebenden UV-Strahler-Röhren 124;
(c) wobei das Oberteil 120 im Verhältnis zu der Liege 110 zum Einsteigen eines Benutzers geöffnet und bei Beginn eines Bräunungsvorgangs geschlossen werden kann; und
(d) vom Benutzer bedienbaren Steuergeräten 130 zur Steuerung der Bedingungen eines Bräunungsvorganges.

Erfindungsgemäß weist das Bräunungsgerät weiter auf
(e) eine Vorrichtung 1 zum Schutz der Augen eines Benutzers vor Strahlung, wie sie von der Erfindung vorgeschlagen wird, die je eine für die Strahlung wenig durchlässige Einrichtung 3 zum Abdecken jeder Augenhöhle des Benutzers und eine Einrichtung 7 zum Befestigen der Vorrichtung 1 am Kopf des Benutzers umfaßt, wobei die Vorrichtung 1 weiter umfaßt:
(f) eine oder mehrere Einrichtung(en) 9, die ein Empfangen optischer Signale von einem Ort ermöglichen, der von der Vorrichtung 1 beabstandet ist, worin die Einrichtung zum Empfangen optischer Signale ein mindestens ein optisches Element umfassendes System zur optischen Übertragung sichtbarer Licht-Signale ist, worin die Einrichtung eine Öffnung in der für Strahlung wenig durchlässigen Einrichtung umfaßt, die die optischen Signale auf das mindestens eine optische Element passieren läßt, das seinerseits die optischen Signale an das Auge weiterleitet, oder worin die Einrichtung ein für sichtbares Licht durchlässiges Sichtfenster der Oberfläche in der für Strahlung wenig durchlässigen Einrichtung umfaßt, das die optischen Signale auf das mindestens eine optische Element passieren läßt, das seinerseits die optischen Signale an das Auge weiterleitet.

Das erfindungsgemäße Bräunungsgerät kann jedes beliebige übliche Bräunungsgerät sein, wie es Fachleuten auf dem vorliegenden Gebiet geläufig ist, mit Ausnahme der erfindungsgemäßen Vorrichtung 1 zum Schutz der Augen eines Benutzers vor Strahlung, insbesondere UV-Strahlung und/oder sichtbarer Strahlung, weiter bevorzugt von Strahlung, wie sie von den UV-Strahler-Röhren des Bräunungsgeräts abgegeben wird.

Ein solches Bräunungsgerät kann entweder den ganzen Körper bräunen oder einzelne Teile davon, beispielsweise das Gesicht, die Arme, die Beine und/oder den Korpus des Benutzers. Weiter umfassen Bräunungsgeräte gemäß der Erfindung sowohl übliche Bräunungsgeräte, in/auf denen der Bräunungsvorgang im Liegen stattfindet, als auch solche, in denen der Bräunungsvorgang im Sitzen, Stehen oder sonstwie stattfindet. Der Begriff "Liege" beschränkt also das Bräunungsgerät nicht auf einen im Liegen stattfindenden Bräunungsvorgang, sondern soll auch den beispielsweise im Stehen stattfindenden Bräunungsvorgang umfassen, und steht dann für im Rücken und/oder seitlich und/oder im vorderen Bereich des Benutzers angeordnete Strahler-Röhren.

In einer bevorzugten Ausführungsform umfasst das Bräunungsgerät 100 zusätzlich (g) eine oder mehrere Einrichtungen, die die Kontrolle des Tragens der Vorrichtung 1 durch den Benutzer durch die Steuergeräte 130 des Bräunungsgeräts 1 ermöglichen.

Dies kann auf an sich beliebige, die Erfindung nicht beschränkende Weise geschehen. Beispielsweise können die Einrichtungen die Kontrolle über die Möglichkeit ausüben, das Bräunungsgerät 100 erst dann einzuschalten, wenn der Benutzer die Vorrichtung 1 ordnungsgemäß trägt. Alternativ oder gleichzeitig kann eine Kontrolle darin bestehen, das Bräunungsgerät 100 dann abzuschalten, wenn der Benutzer die Vorrichtung 1 ablegt, beispielsweise die Vorrichtung ablegt, bevor das Bräunungsgerät auf Veranlassung des Benutzers oder durch Betätigen der entsprechenden Steuergeräte 130 abgeschaltet wird.

In einer weiteren, erfindungsgemäß besonders bevorzugten, weil mit Vorteil verwendbaren Ausführungsform umfasst das Bräunungsgerät 100 zusätzlich eine oder mehrere von dem Gerät beabstandet angeordnete Einrichtungen 140 zum Aussenden von optischen Signalen, die mit der Vorrichtung 1 empfangen und vom Benutzer optisch erfaßt werden können, also beispielsweise gesehen werden können.

In besonders bevorzugten Ausführungsformen kann die mit dem erfindungsgemäßen Bräunungsgerät umfasste Vorrichtung 1 alle Merkmale aufweisen, wie sie oben im Detail für die erfindungsgemäße Vorrichtung 1 erläutert wurden. Aus Gründen der Kürze werden diese Merkmale nicht alle im einzelnen wiederholt. Alle einzelnen, oben beschriebenen Merkmale der Vorrichtung 1, oder auch beliebige Kombinationen der oben beschriebenen Merkmale können jedoch zusammen mit einem Bräunungsgerät 100 gemäß der Erfindung verwirklicht werden und sind daher von der Erfindung umfasst.

Die Erfindung kann benutzt werden für ein Verfahren zum Bräunen zumindest von Teilen des Körpers eines Menschen oder Benutzers. Dies kann das Gesicht/der Kopf, der Oberkörper (gegebenenfalls einschließlich des Kopfes), die Extremitäten (Arme, Beine) oder der gesamte Körper sein.

Es erfolgt das Bräunen mittels Einrichtungen zur Abgabe bräunende Strahlung umfassender Strahlung, wie sie grundsätzlich in diesem Bereich dem Fachmann und dem Benutzer bekannt sind. Die Strahlung wird im natürlichen Bereich von der Sonne, im künstlichen Bereich regelmäßig von UV-Strahlungsquellen wie UV-Strahler-Röhren oder Hochdruckbrennem erzeugt und wird (gefiltert oder ungefiltert; letzteres ist bevorzugt) auf den Körper des Menschen bzw. Benutzers eingestrahlt. Dafür werden bei Bestrahlung mit künstlicher bräunender UV-Strahlung bevorzugt Bräunungsgeräte verwendet, die gewählt sind aus Ganzkörper-Bräunungsgeräten, Gesichts-Bräunungsgeräten oder Bräunungsgeräten für die Extremitäten eines Menschen oder Benutzers.

Es erfolgt das Bräunen nach dem hier vorgeschlagenen Verfahren unter Schützen des Auges/der Augen des Menschen oder Benutzers vor für die Augen schädlichen Komponenten der Strahlung unter gleichzeitigem Empfang optischer Signale mit einer Vorrichtung, wie sie oben im einzelnen anhand bevorzugter Ausführungsformen beschrieben wurde, von einem von der Vorrichtung 1 beabstandeten Ort.

Das Verfahren kann ein Verfahren zur Bestrahlung der Haut für medizinische Zwecke sein, die insbesondere bei UV-Strahlungs-Mangel-Krankheiten oder bei Krankheiten angezeigt sind, für deren Heilung oder Linderung eine UV-Bestrahlung der Haut förderlich ist (z. B. Psoriasis). Alternativ oder gleichzeitig kann das Verfahren ein Verfahren zur Bestrahlung der Haut für kosmetische Zwecke sein, beispielsweise zum Vorbräunen der Haut vor erwarteter intensiver Sonnenbestrahlung (beispielsweise vor dem Verreisen in Gegenden mit stärkerer Sonnenstrahlung als am gewöhnlichen Aufenthaltsort) oder zum Bräunen der Haut aus erwünschten optischen Gründen. Das Bräunungsverfahren kann für jeden beliebigen Zweck eingesetzt werden.

## Patentansprüche

1. Vorrichtung (1) zum Schutz der Augen eines Menschen oder Benutzers vor Strahlung, umfassend zumindest eine für die Strahlung zumindest partiell wenig durchlässige Einrichtung (3) zum Abdecken eines Auges oder beider Augen des Menschen oder Benutzers und eine Einrichtung (7) zum Befestigen der Vorrichtung (1) am Kopf des Menschen oder Benutzers, wobei die Vorrichtung (1) weiter umfaßt:
eine oder mehrere Einrichtung(en) (9), die ein Empfangen optischer Signale von einem Ort ermöglichen, der von der Vorrichtung (1) beabstandet ist,
worin die Einrichtung (9) zum Empfangen optischer Signale ein mindestens ein optisches Element (93) umfassendes System zur optischen Übertragung sichtbarer Licht-Signale ist,
worin die Einrichtung (9) eine Öffnung in der für Strahlung wenig durchlässigen Einrichtung (9) umfaßt, die die optischen Signale auf das mindestens eine optische Element (93) passieren läßt, das seinerseits die optischen Signale an das Auge weiterleitet, oder worin die Einrichtung (9) ein für sichtbares Licht durchlässiges Sichtfenster (97) der Oberfläche in der für Strahlung wenig durchlässigen Einrichtung (9) umfaßt, das die optischen Signale auf das mindestens eine optische Element (93) passieren läßt, das seinerseits die optischen Signale an das Auge weiterleitet.

2. Vorrichtung (1) nach Anspruch 1, umfassend zusätzlich eine zwei Einrichtungen (3) im Gesichtsbereich variabel verbindende Einrichtung (5).

3. Vorrichtung (1) nach einem der Ansprüche 1 oder 2 zum Schutz der Augen eines Menschen oder Benutzers vor natürlicher Strahlung und/oder zum Schutz der Augen eines Menschen oder Benutzers vor künstlicher Strahlung, vorzugsweise vor Strahlung in einem Bräunungsgerät, weiter bevorzugt in einem Bräunungsgerät mit UV-Strahlungsquellen.

4. Vorrichtung (1) nach einem der Ansprüche 1 bis 3, umfassend weiter eine oder mehrere Einrichtungen, die die Kontrolle des Tragens der Vorrichtung (1) durch den Menschen oder Benutzer durch eine Kontroll-Einrichtung, insbesondere einem Bräunungsgerät, ermöglicht.

5. Vorrichtung (1) nach Anspruch 4, worin die Einrichtung(en) gewählt ist/sind aus elektrischer Kontroll-Einrichtung, elektronischer Kontroll-Einrichtung und funkgesteuerter Kontroll-Einrichtung.

6. Vorrichtung (1) nach einem der Ansprüche 1 bis 6, worin die geringe Durchlässigkeit für Strahlung eine geringe Durchlässigkeit für UV-Strahlung ist, vorzugsweise worin die geringe Durchlässigkeit für UV-Strahlung eine Durchlässigkeit im Bereich von 250 bis 320 nm von höchstens 0,1 % der eingestrahlten UV-Strahlung und im Bereich von 320 bis 400 nm von höchstens 1 % der eingestrahlten UV-Strahlung ist, und/oder eine geringe Durchlässigkeit für sichtbare Strahlung ist, vorzugsweise worin die geringe Durchlässigkeit für Strahlung im sichtbaren Bereich eine Durchlässigkeit im Bereich von 400 bis 550 nm von höchstens 5 % der eingestrahlten sichtbaren Strahlung ist.

7. Vorrichtung (1) nach einem der Ansprüche 1 bis 6, worin die Einrichtung (9) zum Empfangen optischer Signale gewählt ist aus Kabelverbindung, Sender/Empfänger, Mikrodisplays und einem oder mehreren gegebenenfalls beweglichen Spiegeln zur Bildung von Mikroarrays.

8. Vorrichtung (1) nach einem der Ansprüche 1 bis 7, worin die Einrichtung (9) zum Empfangen optischer Signale mindestens ein Display, eine Schicht oder einen Film auf der Innenfläche der Einrichtung (3) oder mehrere bewegliche Spiegel zur Bildung von Mikroarrays umfasst, der/die die Wiedergabe optischer Signale in einer für das geschützte Auge sichtbaren Form umfasst/umfassen, vorzugsweise worin die Einrichtungen (9) zum Empfangen optischer Signale ein LCD (911) ist.

9. Vorrichtung (1) nach einem der Ansprüche 1 bis 7, worin das optische Element (93) gewählt ist aus Spiegel und Prisma.

10. Vorrichtung (1) nach einem der Ansprüche 1 bis 6 und 9, worin die optischen Signale von einem optische Signale aussendenden Bildschirm oder Display stammen, der/die außerhalb des Aufenthaltsbereichs des Menschen oder Benutzers angeordnet ist/sind, der sich dem Bräunungsvorgang an oder in einem Bräunungsgerät unterzieht.

11. Vorrichtung (1) nach einem der Ansprüche 1 bis 6 und 9, worin das mindestens eine optische Element (93) im Verhältnis zum Auge des Benutzers so im Lichtweg der optischen Signale angeordnet ist, daß im wesentlichen nur die optischen Signale das Auge erreichen, im wesentlichen jedoch nicht UV-Strahlung oder sichtbare Strahlung, die von UV-Strahlungsquellen stammen.

12. Vorrichtung (1) nach Anspruch 11, worin UV-Strahlung und sichtbare Strahlung, die von UV-Strahlungsquellen stammen, durch einen in die Einrichtung (3) eingebauten Shutter (99) abgeschirmt werden, vorzugsweise worin der Shutter (99) gewählt ist aus optischem Shutter (LCD-Shutter) und mechanischem Shutter.

13. Vorrichtung (1) nach Anspruch 12, worin der Shutter (99) steuerbar ist und in Abhängigkeit von Daten gesteuert wird, die von einem Sensor ermittelt werden, vorzugsweise worin der Sensor die Lage der Vorrichtung (1) in Bezug auf die Lichtquelle und/oder das in die Einrichtung (9) einfallende Licht aus den UV-Strahlungsquellen und/oder eine dem in die Einrichtung (9) einfallenden Licht äquivalente Größe ermittelt, und/oder worin die Steuerung in der Weise erfolgt, daß auf das mit der Vorrichtung (1) geschützte Auge eine Menge an Strahlung fällt, die kleiner oder höchstens gleich der höchstzulässigen Strahlungsmenge ist.

14. Vorrichtung (1) nach einem der Ansprüche 1 bis 13, worin die vom Auge empfangenen optischen Signale gewählt sind aus Fernsehfilm-Signalen, Kinofilm-Signalen, Videofilm-Signalen, CD-Film-Signalen, DVD-Film-Signalen, Internet-Signalen, Musicclip-Signalen, Handy-Bildschirm-Signalen, Videospiel-Signalen, Spiele-Signalen und Spielkonsole-Signalen.

15. Bräunungsgerät (100), umfassend
(a) eine Liege (110) mit einer für bräunende Strahlung im wesentlichen transparenten Liegefläche (112) für den Benutzer und bräunende Strahlung abgebende UV-Strahlungsquellen (114);
(b) ein bewegliches Oberteil (120) mit einer für bräunende Strahlung im wesentlichen transparenten Schutzfläche (122) und bräunende Strahlung abgebenden UV-Strahlungsquellen (124);
(c) wobei das Oberteil (120) im Verhältnis zu der Liege (110) zum Einsteigen eines Benutzers geöffnet und bei Beginn eines Bräunungsvorgangs geschlossen werden kann;
(d) vom Benutzer bedienbaren Steuergeräten (130) zur Steuerung der Bedingungen eines Bräunungsvorganges; und
(e) eine Vorrichtung (1) gemäß Anspruch 1.

16. Bräunungsgerät (100) nach Anspruch 15, umfassend zusätzlich
(f) eine oder mehrere Einrichtungen, die die Kontrolle des Tragens der Vorrichtung (1) durch den Benutzer durch die Steuergeräte (130) des Bräunungsgeräts (1) ermöglichen, vorzugsweise worin die Einrichtungen die Kontrolle über die Möglichkeit ausüben, das Bräunungsgerät (100) erst dann einzuschalten, wenn der Benutzer die Vorrichtung (1) ordnungsgemäß trägt, und/oder das Bräunungsgerät (100) dann abzuschalten, wenn der Benutzer die Vorrichtung (1) ablegt.

17. Bräunungsgerät (100) nach einem der Ansprüche 15 oder 16, umfassend zusätzlich eine oder mehrere von dem Gerät beabstandet angeordnete Einrichtungen (140) zum Aussenden von optischen Signalen, die mit der Vorrichtung (1) empfangen und vom Benutzer optisch erfaßt werden können.

18. Bräunungsgerät (100) nach einem der Ansprüche 15 bis 17, umfassend die Vorrichtung (1) zusätzlich mit einem der Merkmale der Ansprüche 3 bis 14.

## Claims

1. Device (1) for protecting the eyes of a person or user against radiation, comprising at least one arrangement (3) being at least partially little translucent for the radiation for covering one eye or both eyes of the person or user, and an arrangement (7) for attaching the device (1) to the head of a person or user, wherein the device (1) further comprises:
one or several arrangement(s) (9) allowing a receipt of optical signals from a location being spaced from the device (1),
wherein the device (9) for receiving optical signals is a system comprising at least one optical element (93) for optical transmission of visible light signals,
wherein the device (9) comprises an aperture in the arrangement (9) being little translucent for radiation, wherein the opening allows the optical signals to pass to the at least one optical element (93), which on its part further directs the optical signals to the eye, or wherein the device (9) comprises a side window (97) of the surface in the arrangement (9) being little translucent for radiation, wherein the side window (97) is translucent for visible light and allows the optical signals to pass to the at least one optical element (93), which on its part further directs the optical signals to the eye.

2. Device (1) according to claim 1, additionally comprising an arrangement (5) variably connecting both arrangements (3) in the face region.

3. Device (1) according to any of claims 1 or 2 for protecting the eyes of a person or user from natural radiation and/or for protecting the eyes of a person or user from artificial radiation, preferred from radiation in a browning apparatus, in particular in a browning apparatus with UV-radiation sources.

4. Device (1) according to any of claims 1 to 3, further comprising one or several arrangements allowing checking the wearing of the device (1) by the person or user by a checking arrangement, in particular a browning apparatus.

5. Device (1) according to claim 4, wherein the arrangement(s) is / are chosen from electrical checking arrangement, electronical checking arrangement and radio controlled checking arrangement.

6. Device (1) according to any of claims 1 to 6, wherein the low transmittance is a low transmittance for UV-radiation, preferred wherein the low transmittance for UV-radiation is a transmittance in the range of 250 to 320 nm of at most 0,1 % of the irradiated UV-radiation and in the range of 320 to 400 nm of at most 1 % of the irradiated UV-radiation, and/or is a lower transmittance for visible radiation, preferred wherein the low transmittance for radiation in the visible range is a transmittance in the range of 400 to 550 nm of at most 5 % of the irradiated visible radiation.

7. Device (1) according to any of claims 1 to 6, wherein the arrangement (9) for receiving optical signals is chosen from cable connector, sender/receiver, micro displays, and one or several, if necessary, movable mirrors for forming micro arrays.

8. Device (1) according to any one of claims 1 to 7, wherein the device (9) for receiving optical signals comprises at least a display, a layer or a film on the inner face of the arrangement (3), or several movable mirrors for forming micro arrays, comprising reproduction of optical signals in a form visible for the protected eye, preferred
wherein the arrangement (9) for receiving optical signals is a LCD (911).

9. Device (1) according to any of claims 1 to 7, wherein the optical element (93) is chosen from mirror and prism.

10. Device (1) according to any of claims 1 to 6 and 9, wherein the optical signals originate from a monitor or display emitting optical signals, which are located outside the stay area of the person or user undergoing the browning procedure at or in a browning apparatus.

11. Device (1) according to any of claims 1 to 6 and 9 wherein the at least one optical element (93) is arranged in relation to the eye of the user in the light path of the optical signals such that substantially only the optical signals reach the eye, substantially, however, not UV-radiation or a visible radiation, originating from the UV-radiation sources.

12. Device (1) according to claim 11, wherein UV-radiation and visible radiation originating from the UV-radiation sources are shielded by a shutter (99) mounted in the arrangement (3), preferred wherein the shutter (99) is chosen from optical shutter (LCD-shutter) and mechanical shutter.

13. Device (1) according to claim 12, wherein the shutter (99) is controllable and controlled depending on data detected by a sensor, preferred wherein the sensor detects the location of the device (1) with respect to the light source and / or the light shining in the arrangement (9) from the UV-radiation sources and / or a variable being equivalent to the light shining in the arrangement (9), and/or wherein the controlling is performed in such a manner that the eye protected by the device (1) receives such an amount of radiation, which is smaller or at most equal to the maximum allowable amount of radiation.

14. Device (1) according to any of claims 1 to 13, wherein the optical signals received by the eye are chosen from television film signals, movie film signals, video film signals, CD-film signals, DVD-film signals, internet signals, music clip signals, mobile phone display signals, video game signals, game signals and paddle signals.

15. Browning apparatus (100) comprising
(a) a bed (110) having a rest face (112) substantially transparent for browning radiation for the user, and UV-radiation sources (114) emitting browning radiation:
(b) a movable top part (120) having a protection face (122) substantially transparent for browning radiation, and UV-radiation sources (124) emitting browning radiation;
(c) wherein the top part (120) can be opened in relation to the bed (110) for the entering user, and can be closed at the beginning of the browning procedure;
(d) control apparatuses (130) operable by the user for controlling the conditions of a browning procedure; and
(e) a device (1) according to claim 1.

16. Browning apparatus (100) according to claim 15, additionally comprising
(f) one or several arrangement(s) allowing the checking of wearing the device (1) by the user by the control apparatuses (130) of the browning apparatus (1), preferred wherein the arrangements perform the checking via the possibility to switch on the browning apparatus (100) not until when the user is properly wearing the device (1), and / or to switch off the browning apparatus (100) when the user is putting off the device (1).

17. Browning apparatus (100) according to any of claims 15 or 16, additionally comprising one or several arrangements (140) spaced from the apparatus for emitting optical signals received by the device (1) and which can be optically realized by the user.

18. Browning apparatus (100) according to any of claims 15 to 17, comprising the device (1) in addition with one of the features according to claims 3 to 14.

## Revendications

1. Dispositif (1) pour protéger les yeux d'une personne ou d'un utilisateur contre un rayonnement, comprenant au moins un organe (3) qui est au moins en partie peu transparent au rayonnement pour recouvrir un oeil ou les deux yeux de la personne ou de l'utilisateur et un organe (7) pour fixer le dispositif (1) sur la tête de la personne ou de l'utilisateur, le dispositif (1) comprenant en plus :
un ou plusieurs agencements (9) de réception de signaux optiques depuis un lieu éloigné du dispositif (1),
dans lequel l'agencement (9) de réception de signaux optiques est un système qui comprend au moins un élément optique (93) pour la transmission optique de signaux lumineux visibles,
dans lequel l'agencement (9) comprend une ouverture dans l'organe peu transparent au rayonnement (9) qui laisse passer les signaux optiques vers ledit au moins un élément optique (93), lequel à son tour transmet les signaux optiques à l'oeil, ou dans lequel l'agencement (9) comprend une fenêtre de visualisation transparente à la lumière visible (97) dans la surface de l'agencement peu transparent au rayonnement (9) qui laisse passer les signaux optiques vers ledit au moins un élément optique (93), lequel à son tour transmet les signaux optiques à l'oeil.

2. Dispositif (1) selon la revendication 1, comprenant en plus un élément (5) qui relie de manière variable deux organes (3) dans la zone du visage.

3. Dispositif (1) selon l'une des revendications 1 ou 2, destiné à protéger les yeux d'une personne ou d'un utilisateur contre un rayonnement naturel et/ou à protéger les yeux d'une personne ou d'un utilisateur contre un rayonnement artificiel, de préférence contre le rayonnement dans un appareil de bronzage, de façon particulièrement préférée dans un appareil de bronzage équipé de sources de rayonnement UV.

4. Dispositif (1) selon l'une des revendications 1 à 3, comprenant en plus un ou plusieurs éléments permettant de contrôler le port du dispositif (1) par la personne ou l'utilisateur au moyen d'un appareil de contrôle, en particulier d'un appareil de bronzage.

5. Dispositif (1) selon la revendication 4, dans lequel le ou les appareils sont choisis parmi un appareil de contrôle électrique, un appareil de contrôle électronique et un appareil de contrôle radiocommandé.

6. Dispositif (1) selon l'une des revendications 1 à 6, dans lequel la faible transparence au rayonnement est une faible transparence au rayonnement UV dans laquelle ladite faible transparence au rayonnement UV est de préférence une transparence au rayonnement UV au maximum égale à 0,1% du rayonnement UV incident dans la plage de 250 à 320 nm et une transparence au rayonnement UV au maximum égale à 1% du rayonnement UV incident dans la plage de 320 à 400 nm, et/ou une transparence au rayonnement visible dans laquelle ladite transparence au rayonnement visible est de préférence une transparence au rayonnement visible au maximum égale à 5% du rayonnement visible incident dans la plage de 400 à 550 nm.

7. Dispositif (1) selon l'une des revendications 1 à 6, dans lequel l'agencement (9) de réception de signaux optiques est choisi parmi une liaison par câble, un émetteur/ récepteur, des micro-afficheurs et un ou plusieurs miroirs le cas échéant mobiles pour former des micro-réseaux.

8. Dispositif (1) selon l'une des revendications 1 à 7, dans lequel l'agencement (9) de réception de signaux optiques comprend au moins un afficheur, une couche ou un film sur la surface intérieure de l'organe (3) ou plusieurs miroirs mobiles pour former des micro-réseaux qui comprennent la restitution de signaux optiques sous une forme visible par l'oeil protégé, de préférence dans lequel l'agencement (9) de réception de signaux optiques est un écran LCD (911).

9. Dispositif (1) selon l'une des revendications 1 à 7, dans lequel l'élément optique (93) est choisi parmi un miroir et un prisme.

10. Dispositif (1) selon l'une des revendications 1 à 6 et 9, dans lequel les signaux optiques proviennent d'un écran ou afficheur envoyant des signaux optiques qui est disposé à l'extérieur de la zone dans laquelle se trouve la personne ou l'utilisateur qui s'expose au processus de bronzage sur ou dans un appareil de bronzage.

11. Dispositif (1) selon l'une des revendications 1 à 6 et 9, dans lequel ledit au moins un élément optique (93) est disposé dans le trajet lumineux des signaux optiques, par rapport à l'oeil de l'utilisateur, de façon qu'il n'y ait pratiquement que les seuls signaux optiques qui parviennent à l'oeil mais pratiquement pas de rayonnement UV ou de rayonnement visible provenant de sources de rayonnement UV.

12. Dispositif (1) selon la revendication 11, dans lequel le rayonnement UV et le rayonnement visible provenant de sources de rayonnement UV sont arrêtés par un obturateur (99) incorporé dans l'organe (3), l'obturateur (99) étant de préférence choisi parmi un obturateur optique (obturateur LCD) et un obturateur mécanique.

13. Dispositif (1) selon la revendication 12, dans lequel l'obturateur (99) peut être commandé et est commandé en fonction de données déterminées par un capteur, dans lequel le capteur détecte de préférence la position du dispositif (1) par rapport à la source lumineuse et/ou la lumière incidente à l'agencement (9) venant des sources de rayonnement UV et/ou une grandeur équivalente à la lumière incidente à l'agencement (9), et/ou dans lequel la commande se fait de telle manière que l'oeil protégé par le dispositif (1) reçoit une quantité de rayonnement qui est inférieure ou au maximum égale à la quantité de rayonnement maximale admissible.

14. Dispositif (1) selon l'une des revendications 1 à 13, dans lequel les signaux optiques reçus par l'oeil sont choisis parmi des signaux de téléfilms, des signaux de films cinématographiques, des signaux de films vidéo, des signaux de films sur CD, des signaux de films sur DVD, des signaux Internet, des signaux de clips musicaux, des signaux d'écrans de téléphones portables, des signaux de jeux vidéo, des signaux de jeux et des signaux de consoles de jeu.

15. Appareil de bronzage (100), comprenant
(a) une couchette (110) avec une surface de couchette (112) pratiquement transparente au rayonnement bronzant pour l'utilisateur ainsi que des sources de rayonnement UV délivrant un rayonnement bronzant (114) ;
(b) une partie supérieure mobile (120) avec une surface de protection pratiquement transparente au rayonnement bronzant (122) ainsi que des sources de rayonnement UV délivrant un rayonnement bronzant (124) ;
(c) dans lequel la partie supérieure (120) peut être ouverte par rapport à la couchette (110) pour permettre à un utilisateur de s'installer et fermée au début d'une séance de bronzage ;
(d) des appareils de commandes actionnables par l'utilisateur (130) pour commander les conditions d'une séance de bronzage ; et
(e) un dispositif (1) selon la revendication 1.

16. Appareil de bronzage (100) selon la revendication 15, comprenant en plus:
(f) un ou plusieurs moyens permettant à l'utilisateur de contrôler le port du dispositif (1) au moyen des appareils de commande (130) de l'appareil de bronzage (1), de préférence dans lequel les moyens exercent le contrôle grâce à la possibilité de ne mettre l'appareil de bronzage (100) en marche que lorsque l'utilisateur porte correctement le dispositif (1) et/ou de stopper l'appareil de bronzage (100) lorsque l'utilisateur enlève le dispositif (1).

17. Appareil de bronzage (100) selon l'une des revendications 15 ou 16, comprenant en plus un ou plusieurs moyens (140) disposés à une certaine distance de l'appareil pour envoyer des signaux optiques qui peuvent être reçues au moyen du dispositif (1) et détectés optiquement par l'utilisateur.

18. Appareil de bronzage (100) selon l'une des revendications 15 à 17, comprenant le dispositif (1) avec en plus l'un des attributs des revendications 3 à 14.
